# EUROPÄISCHE PATENTSCHRIFT

(11) **EP 1 806 126 B1**
(45) Veröffentlichungstag und Bekanntmachung des Hinweises auf die Patenterteilung: **04.06.2014**
(21) Anmeldenummer: 06023872.2
(22) Anmeldetag: 17.11.2006
(51) Int. Cl.: A61K 8/66, A61K 8/34, A61Q 11/00

(54) **Mund- und Zahnpflege und -reinigungsmittel mit Enzym(en)**
Dental preparations comprising enzymes
Preparations dentaires contenant des enzymes

(30) Priorität: 06.01.2006 DE 102006001148
(43) Veröffentlichungstag der Anmeldung: 11.07.2007
(73) Patentinhaber: Henkel AG & Co. KGaA, 40589 Düsseldorf (DE)
(72) Erfinder: Barth, Adolf Peter, 42781 Haan (DE); Schmidt, Irmgard, 42697 Solingen (DE); Maurer, Karl-Heinz, 40699 Erkrath (DE); Weber, Thomas, 40589 Düsseldorf (DE); Spitz, Astrid, 47441 Moers (DE); Janssen, Frank, 41470 Neuss (DE); Breves, Roland, 40822 Mettmann (DE); Bernecker, Ullrich, 52074 Aachen (DE); Howorka, Wilfried, 40597 Düsseldorf (DE)

(56) Entgegenhaltungen:
- WO-A-99/06573
- GB-A- 1 222 197
- JP-A- 4 316 512
- US-A- 5 470 725
- US-A1- 2002 086 039
- US-A1- 2003 211 053
- US-B1- 6 692 726

## Beschreibung

Die Erfindung betrifft Zubereitungen zur Mund- und Zahnpflege- und -reinigung, die β-Glucanase aus Bacillus amyloliquefaciens, enthalten.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Neben der Reinigung der Zähne erwartet der Verbraucher von den gattungsgemäßen Produkten auch eine Pflege der Zähne und der Mundhöhle. So sind insbesondere ein "sauberes" Gefühl, d.h. eine glatte und glänzende Zahnoberfläche sowie ein frisches Gefühl im Mund wesentliche Aspekte für den Kaufanreiz von Zubereitungen zur Mund- und Zahnpflege- und -reinigung. Ein erfolgreiches Mittel der gattungsgemäßen Art sollte daher die Zähne gründlich reinigen, ohne den Zahn oder die Zahnoberfläche zu schädigen und gleichzeitig Mundgeruch verringern und/oder verhindern.

In unzugänglichen Bereichen des Mundes wie beispielsweise in Zahnzwischenräumen oder dem Bereich der hinteren Backenzähne ist die herkömmliche mechanische Reinigung mit der Zahnbürste oft nicht vollständig erfolgreich, insbesondere dann, wenn die Putzdauer nicht ausreichend ist. Dies hat zur Folge, dass der Zahnbelag in diesen Bereichen nicht vollständig entfernt wird und sich Mikroorganismen wieder auf den Zähnen ansiedeln können, was zur Plaqueneubildung führt.

Es ist grundsätzlich seit langem bekannt, Enzyme zur Lösung dieser Probleme einzusetzen, wobei die Enzyme gezielt die Bestandteile der Plaque abbauen und die Plaque entfernen, die Zähne und das Zahnfleisch aber nicht schädigen.

So werden in US1232627 Zahnpasten beschrieben, die eine Protease und eine Amylase aus Bacillus subtilis enthalten.

In DE1944308 werden Zahn- und Mundpflegemittel beschrieben, die Enzymkomplexe und/oder Enzymgemische aus Dextranasen, weiteren Carbohydrasen und/oder Proteasen enthalten. Als Carbohydrasen werden alpha- und/oder beta-Amylasen sowie Glucoamylasen genannt. Als Proteasen werden Pepsin, Trypsin, Papain, Bromelin und Ficin genannt.

In DE2000820 wird eine Mundpflege-Zubereitung beschrieben, die eine neutrale Protease sowie ggf. geringe Mengen an Amylase oder alkalischer Protease enthält. Den Beispielen ist zu entnehmen, dass die neutrale Protease aus einem Bacillus subtilis-Stamm isoliert wurde.

In DE2408997 wird ein Mund- und Zahnreinigungsmittel beschrieben, das ein Fructan abbauendes Enzym sowie gegebenenfalls zusätzlich Dextranase, Amylase, Protease oder Lipase enthält.

In CA959764 wird ein Mittel zur oralen Pflege beschrieben, das Carbohydrasen, Lipasen oder Proteasen ausgewählt aus Alcalase, Maxatase, Ficin, Bromelin, Trypsin, Chymotrypsin, Protease AP-1 00, M-Zyme und Rhozyme P-11 enthält.

In US4082841 wird eine Zahnpaste beschrieben, die Enzyme ausgewählt aus Proteasen, Carbohydrasen und Lipasen enthalten kann. Als Proteasen genannt werden Alcalase, Maxatase, Protease AP-100, Prolase 300 trypsin, Chymotrypsin, Papain, Ficin, Bromelin, M-Zyme und Rhozyme P-11. Als Carbohydrasen werden genannt Dextranase, Cellulase, Cellzyme A (alpha-beta amylase), Mylase 100, Mylase W, Amylase L-661 und Fungal Amylase. Als Lipasen werden genannt Lipase 4000, Lipase B, Lipase 448, gastrische Lipase, pankreatische Lipase und pflanzliche Lipase.

In US4335101 wird ein Mittel zur Zahnpflege beansprucht, das Dextranase sowie ein Enzym zur Spaltung von durch Streptococcen produziertem dentalem Plaque-Glucan mit größtenteils alpha-1,3-glucosidischer Bindung enthält.

In WO95/31533 wird die Verwendung einer endo-beta-Glucanase in Mitteln zur Entfernung von Plaque beschrieben. Als Quellen für die Glucanase werden Trichoderma, Saccharomyces, Aspergillus, Penicillium, Fusarium, Humicola, Acremonium, Botrytis und Sclerotium genannt.

In EP632828 wird eine Endo-beta-1,4-Glucanase aus Aspergillus offenbart.

In WO95/31533 wird die Verwendung einer Dextranase oder 1,3-Glucanase in Mitteln zur oralen Pflege beschrieben. Eine Quelle für die 1,3-Glucanase wird nicht genannt.

US 6 692 726 B1 (MORGAN ANDRE M [US] ET AL) 17. Februar 2004 offenbart (Spalte 3, Zeile 16) das für die orale kosmetische Anwendung geeignete Enzym Glucanase. Ferner werden Putzkörper beschrieben (Spalte 4, Zeile 40-67).

Erfindungsgemäß wurde nun überraschenderweise gefunden, dass Enzyme ausgewählt aus β-Glucanase aus Bacillus, insbesondere aus Bacillus amyloliquefaciens, Protease B7020 aus Aspergillus, insbesondere aus Aspergillus oryzae, Neutrale Protease aus Aspergillus, insbesondere aus Aspergillus oryzeae, Corolase aus Aspergillus, insbesondere aus Apergillus sojae und α-Amylase aus Bacillus, insbesondere aus Bacillus subtilis, in ganz besonderer Weise für den Einsatz in Mitteln zur oralen Pflege geeignet sind.

Ein erster Gegenstand der vorliegenden Erfindung sind daher Mund- und Zahnpflege- und -reinigungsmittel, enthaltend mindestens das Enzym β-Glucanase aus Bacillus amyloliquefaqciens, und 1-30% Putzkörper.

In einer bevorzugten Ausführungsform wird hierbei nur das genannte Enzym eingesetzt, wobei es sich um das einzige im Mittel enthaltene Enzym handelt. Es handelt sich hierbei also um Mittel, die als einziges enthaltenes Enzym nur β-Glucanase aus Bacillus amyloliquefaqciens.

In einer weiteren bevorzugten Ausführungsform wird eine Kombination aus mehreren der genannten Enzyme eingesetzt. Besonders bevorzugt wird hierbei eine Mischung aus β-Glucanase aus Bacillus amyloliquefaciens mit jeweils genau einem der genannten Enzyme eingesetzt, wobei es sich um die beiden einzigen im Mittel enthaltenen Enzyme handelt.

Des weiteren kann zusätzlich mindestens ein weiteres beliebiges Enzym ausgewählt aus der Gruppe der Proteasen, der Amylasen und der Hemicellulasen, beispielsweise Papain oder eine endo-Xylanase, in dem erfindungsgemäßen Mittel enthalten sein.

Mund- und Zahnpflegemittel sowie Mund- und Zahnreinigungsmittel im Sinne der Erfindung sind insbesondere Mund- und Zahnpulver, Mund- und Zahnpasten, flüssige Mund- und Zahncremes, Mund- und Zahngele, Mundwässer und Kaugummis. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden. Unter Mund- und Zahnpflege- und -reinigungsmittel sind erfindungsgemäß des weiteren, in einer weniger bevorzugten Ausführungsform, auch Zahnprothesenpflegemittel zu verstehen, insbesondere Gebissreiniger und Prothesenhaftcremes.

Die erfindungsgemäß geeignete β-Glucanase aus B. amyloliquefaciens wird in der IUBMB Enzym-Nomenklatur mit der Nummer EC 3.2.1.6 bezeichnet, der empfohlene Name für diese Enzyme ist 1,3(4)-b-Glucanase. Andere Namen sind: endo-1,3-b-D-Glucanase; Laminarinase; Laminaranase; b-1.3-Glucanase; b-1,3-1,4-Glucanase; endo-1,3-b-Glucanase; endo-b-1,3(4)-Glucanase; endo-b-1,3-1,4-Glucanase; endo-b-(1→3)-D-Glucanase; endo-1,3-1,4-b-D-Glucanase; endo-b-(1-3)-D-Glucanase; endo-b-1,3-Glucanase IV oder endo-1,3-b-D-Glucanase der systematische Name ist 1,3-(1,3;1,4)-b-D-Glucan 3(4)-Glucanohydrolase. Vorzugsweise handelt es sich bei der β-Glucanase um ein Enzym mit einer Sequenz gemäß Swiss-Prot: Q8GMY0, EMBL: AAN64132, EMBL (DNA): AF546871.

Die erfindungsgemäß geeignete neutrale Protease aus Aspergillus oryzae wird in der der IUBMB Enzym-Nomenklatur mit der Nummer EC 3.4.24.28 bezeichnet.

Die erfindungsgemäß geeignete Corolase aus Aspergillus sojae wird in der IUBMB-Nomenklatur mit der Nummer EC 3.4.21.15 bzw. 3.4.21.63 bezeichnet.

Die erfindungsgemäß geeignete α-Amylase aus B. subtilis wird gemäß der IUBMB-Nomenklatur mit der Nummer EC 3.2.1.1 bezeichnet. Vorzugsweise handelt es sich bei der α-Amylase um ein Enzym mit einer Sequenz gemäß Swiss-Prot: P00691, EMBL (DNA): Z99105.

Die Enzymaktivitäten der in den erfindungsgemäßen Mund- und Zahnpflege- und -reinigungsmittel eingesetzten Enzyme kann von Enzym zu Enzym variieren. Üblicherweise beträgt die Enzymaktivität pro Gramm des erfindungsgemäßen Mund- und Zahnpflege- und -reinigungsmittels zwischen 0,1 und 1500 U (µmol/min).

Erfindungsgemäße Mund- und Zahnpflege- und -reinigungsmittel sind vorzugsweise dadurch gekennzeichnet, dass die Enzymaktivität pro Gramm Mund- und Zahnpflege- und -reinigungsmittel von 0,05 bis 1500 U, vorzugsweise von 0,1 bis 1200 U, besonders bevorzugt von 0,25 bis 1000 U und insbesondere von 0,5 bis 500 U beträgt.

### Besonders bevorzugt sind

- für die beta-Glucanase Werte von 0,1 bis 100 U, vorzugsweise von 0,25 bis 50 U, besonders bevorzugt von 0,5 bis 25 U und insbesondere von 0,5 bis 5 U pro Gramm des erfindungsgemäßen Mund- und Zahnpflege- und -reinigungsmittels;
- für die Proteasen Werte von 0,1 bis 100 U, vorzugsweise von 0,25 bis 50 U, besonders bevorzugt von 0,5 bis 25 U und insbesondere von 0,5 bis 5 U pro Gramm des erfindungsgemäßen Mund- und Zahnpflege- und -reinigungsmittels;
- für die Corolase Werte von 0,1 bis 100 U, vorzugsweise von 0,25 bis 50 U, besonders bevorzugt von 0,5 bis 25 U und insbesondere von 0,5 bis 5 U pro Gramm des erfindungsgemäßen Mund- und Zahnpflege- und -reinigungsmittels;
- für die Amylase Werte von 10 bis 1000 U, vorzugsweise von 50 bis 700 U, besonders bevorzugt von 100 bis 500 U und insbesondere von 100 bis 300 U pro Gramm des erfindungsgemäßen Mund- und Zahnpflege- und -reinigungsmittels.

Die Enzymaktivitäten werden im Rahmen der vorliegenden Erfindung bei 25°C, pH = 7,0 in einem 100 mM Kalium-Phosphat-Puffer gemessen; für die Aktivitätsbestimmung der Proteasen und der Corolase findet die AAPF-Methode, für die Aktivitätsbestimmung der alpha-Amylase die DNS-Methode und für die Aktivitätsbestimmung der beta-Glucanase die PAHBAH-Methode Anwendung.

Die erfindungsgemäßen Mund- und Zahnpflege- und -reinigungsmittel können zusätzlich weitere Inhaltsstoffe von Mundreinigungsmitteln, Mundpflegemitteln, Zahnreinigungsmitteln und/oder Zahnpflegemitteln enthalten. Die bevorzugten weiteren Inhaltsstoffe werden nachstehend beschrieben.

Die erfindungsgemäßen Mund- und Zahnpflege- und -reinigungsmittel enthalten vorzugsweise als weiteren wesentlichen Inhaltsstoff Wasser. Das in erfindungsgemäßen Mund- und Zahnpflege- und -reinigungsmitteln enthaltene Wasser kann Leitungswasser sein, dessen Härtegrad je nach Herstellungsort bzw. Quelle des Wassers variieren kann. Möglich und bevorzugt ist es jedoch, Wasser mit Härtegraden zwischen 0 und 20°dH, vorzugsweise zwischen 1 und 16°dH einzusetzen. Besonders bevorzugt ist der Einsatz von technisch vollentsalztem Wasser ("Wasser VE"), das mit Hilfe von lonenaustauschern weitgehend von Salzen befreit wurde.

Als Feuchthaltemittel können die erfindungsgemäßen Mittel z.B. Glycerin, Sorbit, Xylit, Propylenglykole, Polyethylenglykole oder Gemische dieser Polyole, insbesondere solche Polyethylenglykole mit Molekulargewichten von 200 - 2000 g/mol, vorzugsweise von 200 - 1000 g/mol, enthalten.

Die erfindungsgemäßen Mittel, insbesondere die Zahnpasten oder flüssigen Zahncremes enthalten in einer bevorzugten Ausführungsform ein oder mehrere Poliermittel, üblicherweise in einer Gesamtmenge von 5 bis 50 Gew.-%.

Als Poliermittel eignen sich prinzipiell alle für Zahnpasten bekannten Reibkörper, insbesondere solche, die keine Calciumionen enthalten. Bevorzugt geeignete Poliermittelkomponenten sind daher Kieselsäuren, Aluminiumhydroxid, Aluminiumoxid, Natriumaluminiumsilikate, organische Polymere oder Gemische solcher Reibkörper.

Calciumhaltige Polierkomponenten wie z.B. Kreide, Calciumpyrophosphat, Dicalcium-phosphat-dihydrat können aber in Mengen bis zu 5 Gew.-% enthalten sein.

Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wässrigen Mineralsäuren unter Ausbildung eines Hydrosols, Alterung zum Hydrogel, Waschen und Trocknen hergestellt. Erfolgt die Trocknung unter schonenden Bedingungen auf Wassergehalt von 15 bis 35 Gew.-%, so werden die sogenannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m2/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident12 DS (DEGUSSA) erhältlich.

Andere Fällungskieselsäuren dieser Art sind Sident 8 (DEGUSSA) und Sorbosil AC 39 (Crosfield Chemicals). Diese Kieselsäuren zeichnen sich durch eine geringere Verdickungswirkung und eine etwas höhere mittlere Teilchengröße von 8 -14 µm bei einer spezifischen Oberfläche von 40 - 75 m2/g (nach BET) aus und eignen sich besonders gut für flüssige Zahncremes. Diese sollten eine Viskosität (25°C, Scherrate D = 10 s-1) von 10 - 100 Pa.s aufweisen.

Zahnpasten, die eine deutlich höhere Viskosität von mehr als 100 Pa.s (25° C, D = 10 s-1) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, sogenannte Verdickungskieselsäuren mit einer BET-Oberfläche von 150 -250 m2/g zu, z.B. die Handelsprodukte Sipernat 22 LS oder Sipernat 320 DS.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend enthalten erfindungsgemäße Mund- und Zahnpflege- und - reinigungsmittel zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, in Mengen von 1 bis 30 Gew.%, besonders bevorzugt von 2,5 bis 25 Gew.% und insbesondere von 5 bis 20 Gew.%, jeweils bezogen auf das gesamte Mittel.

Die erfindungsgemäßen Mund- und Zahnpflege- und reinigungsmittel, insbesondere die Zahnpasten, können z. B. auch antimikrobielle Stoffe als Konservierungsmittel oder als Antiplaque-Wirkstoffe enthalten. Derartige Stoffe können z. B. ausgewählt sein aus p-Hydroxybenzoesäuremethyl-, -ethyl- oder propylester, Natriumsorbat, Natriumbenzoat, Bromchlorophen, Triclosan, Phenyl-Salicylsäureester, Biguanide, z. B. Chlorhexidin und Thymol. Diese Stoffe sind in den erfindungsgemäßen Mitteln vorzugsweise in Mengen von 0,1 bis 5 Gew.%, besonders bevorzugt von 0,25 bis 2,5 Gew.% und insbesondere von 0,5 bis 1,5 Gew.%, jeweils bezogen auf das gesamte Mittel, enthalten.

Die Mund- und Zahnpflege- und reinigungsmittel, insbesondere die Zahnpasten, können des weiteren auch Substanzen enthalten, die gegen Zahnstein wirksam sind. Derartige Substanzen können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P₂O₇, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können. Erfindungsgemäß bevorzugte Mund- und Zahnpflege- und -reinigungsmittel sind dadurch gekennzeichnet, daß sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Eine weitere bevorzugte Gruppe von Inhaltsstoffen, die in den erfindungsgemäßen Mitteln enthalten sein kann, sind Antikaries-Wirkstoffe. Diese können beispielsweise aus organischen oder anorganischen Fluoriden ausgewählt sein, z. B. aus Natriumfluorid, Kaliumfluorid, Natriummonofluorphosphat, Natriumfluorosilikat, Zinkfluorid und Zinn-(II)-fluorid. Bevorzugt sollte eine Menge von 0,01 - 0,5 Gew.-% Fluor in Form der genannten Verbindungen enthalten sein.

Die erfindungsgemäßen Mittel, insbesondere die Zahnpasten, können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.

Als nicht-kationische, bakterizide Komponente eignen sich z.B. Phenole, Resorcine, Bisphenole, Salicylanilide und deren halogenierte Derivate, halogenierte Carbanilide und p-Hydroxybenzoesäureester. Besonders bevorzugte antimikrobielle Komponenten sind halogenierte Diphenylether, z.B. 2,4-Dichlor-2'-hydroxydiphenylether, 4,4'-Dichlor-2'-hydroxydiphenylether, 2,4,4'-Tribrom-2'-hydroxydiphenylether und 2,4,4'-Trichlor-2'-hydroxydiphenylether (Triclosan). Sie werden bevorzugt in Mengen von 0,01 - 1 Gew.-% in die erfindungsgemäßen Zahnpflegemittel eingesetzt. Besonders bevorzugt wird Triclosan in einer Menge von 0,01 - 0,3 Gew.-% eingesetzt.

D-Panthenol D-(+)-2,4-Dihydroxy-N-(3-hydroxypropyl)-3,3-dimethyl-butyramid zeigt eine der Pantothensäure entsprechende biologische Aktivität. Die Pantothensäure (R-(+)-N-(2,4-Dihydroxy-3,3-dimethylbutyryl-β-alanin) ist eine Vorstufe in der Biosynthese des Coenzyms A und wird zum Vitamin-B-Komplex (B3) gezählt. Diese Stoffe sind dafür bekannt, daß sie die Wundheilung fördern und eine günstige Wirkung auf die Haut haben. Sie sind daher auch gelegentlich in Zahnpasten beschrieben worden. Die erfindungsgemäßen Zahnpflegemittel enthalten bevorzugt 0,05 - 5 Gew.-% Panthenol oder ein Salz der Pantothensäure.

Retinol (3,7-Dimethyl-9-(2,6,6-trimethyl-1-cyclohexenyl)-2,4,6,8-nonatetraen-1-ol ist der internationale Freiname für Vitamin A1. Anstelle des Retinols kann auch eines seiner Derivate mit ähnlicher biologischer Wirkung, z.B. ein Ester oder die Retinoesäure (Tretinoin), eines ihrer Salze oder ihre Ester verwendet werden. Bevorzugt wird ein Retinol-Ester, insbesondere ein Fettsäureester einer Fettsäure mit 12 - 22 C-Atomen verwendet. Besonders bevorzugt ist Retinol-Palmitat geeignet. Bei Verwendung eines Retinol-Esters, z.B. Retinol-Palmitat mit einer Aktivität von 1,7 · 10⁶ I.E. pro g ist eine Menge von 0,001 bis 0,1 Gew.-% bevorzugt. Bei Verwendung anderer Retinol-Derivate empfiehlt sich eine Einsatzmenge, die einer Konzentration von 10³ bis 10⁶ I.E. (Internationale Einheiten) pro 100 g entspricht.
Bevorzugte Zahnpflegemittel gemäß der vorliegenden Erfindung enthalten neben Poliermitteln, Fluorverbindungen, Feuchthaltemitteln und Bindemitteln bevorzugt

| | |
|---|---|
| 0,01 - 1 | Gew.-% eines halogenierten Diphenylethers |
| 0,05 - 5 | Gew.-% Panthenol oder ein Salz der Pantothensäure und |
| 0,01 - 0,1 | Gew.-% eines Retinol-Esters, bevorzugt Retinol-Palmitat. |

Als Bindemittel bzw. Konsistenzregler dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Auch oberflächenaktive Substanzen können in den Zahnpasten zur Unterstützung der Reinigungswirkung und gewünschtenfalls auch zur Entwicklung von Schaum beim Zähnebürsten sowie zur Stabilisierung der Polierkörperdispersion im Träger vorzugsweise in einer Menge von 0,1-5 Gew.-% enthalten sein.

Als Tenside sind dabei insbesondere Alkyl- und/oder Alkenyl-(oligo)-glycoside einsetzbar. Ihre Herstellung und Verwendung als oberflächenaktive Stoffe sind beispielsweise aus US-A-3 839 318, US-A-3 707 535, US-A-3 547 828 DE-A-19 43 689, DE-A-20 36 472 und DE-A-30 01 064 sowie EP-A-77 167 bekannt. Bezüglich des Glycosidrestes gilt, daß sowohl Monoglycoside (x = 1), bei denen ein Pentose- oder Hexoserest glycosidisch an einen primären Alkohol mit 4 bis 16 C-Atomen gebunden ist, als auch oligomere Glycoside mit einem Oligomerisationsgrad x bis 10 geeignet sind. Der Oligomerisationsgrad ist dabei ein statistischer Mittelwert, dem eine für solche technischen Produkte übliche Homologenverteilung zugrunde liegt.

Bevorzugt eignet sich als Alkyl- und/oder Alkenyl-(oligo)-glycosid ein Alkyl- und/oder Alkenyl-(oligo)-glucosid der Formel RO(C₆H₁₀O)ₓ-H, in der R eine Alkyl- und/oder Alkenyl-gruppe mit 8 bis 14 C-Atomen ist und x einen Mittelwert von 1 bis 4 hat. Besonders bevorzugt sind Alkyloligoglucoside auf Basis von gehärtetem C_{12/14}-Kokosalkohol mit einem DP von 1 bis 3. Das Alkyl- und/oder Alkenyl-glycosid-Tensid kann sehr sparsam verwendet werden, wobei bereits Mengen von 0,005 bis 1 Gew.-% ausreichend sind.

Außer den genannten Alkylglucosid-Tensiden können auch andere nichtionische, ampholytische und kationische Tenside enthalten sein, als da beispielsweise sind: Fettalkoholpolyglycolethersulfate, Monoglyceridsulfate, Monoglyceridethersulfate, Mono- und/oder Dialkylsulfosuccinate, Fettsäureisethionate, Fettsäuresarcosinate, Fettsäuretauride, Fettsäureglutamate, Ethercarbonsäuren, Fettsäureglucamide, Alkylamido-betaine und/oder Proteinfettsäurekondensate, letztere vorzugsweise auf Basis von Weizenproteinen. Insbesondere zur Solubilisierung der meist wasserunlöslichen Aromaöle kann ein nichtionogener Lösungsvermittler aus der Gruppe der oberflächenaktiven Verbindungen erforderlich sein. Besonders geeignet für diesen Zweck sind z.B. oxethylierte Fettsäureglyceride, oxethylierte Fettsäuresorbitanpartialester oder Fettsäurepartialester von Glycerin- oder Sorbitan-Oxethylaten. Lösungsvermittler aus der Gruppe der oxethylierten Fettsäureglyceride umfassen vor allem Anlagerungsprodukte von 20 bis 60 Mol Ethylenoxid an Mono- und Diglyceride von linearen Fettsäuren mit 12 bis 18 C-Atomen oder an Triglyceride von Hydroxyfettsäuren wie Oxystearinsäure oder Ricinolsäure. Weitere geeignete Lösungsvermittler sind oxethylierte Fettsäuresorbitanpartialester; das sind bevorzugt Anlagerungsprodukte von 20 bis 60 Mol Ethylenoxid an Sorbitanmonoester und Sorbitandiester von Fettsäuren mit 12 bis 18 C-Atomen. Ebenfalls geeignete Lösungsvermittler sind Fettsäurepartialester von Glycerin- oder Sorbitan-Oxethylaten; das sind bevorzugt Mono- und Diester von C₁₂-C₁₈-Fettsäuren und Anlagerungsprodukten von 20 bis 60 Mol Ethylenoxid an 1 Mol Glycerin oder an 1 Mol Sorbit.

Die erfindungsgemäßen Mund-, Zahn- und/oder Zahnprothesenpflegemittel enthalten bevorzugt als Lösungsvermittler für gegebenenfalls enthaltene Aromaöle Anlagerungsprodukte von 20 bis 60 Mol Ethylenoxid an gehärtetes oder ungehärtetes Rizinusöl (d.h. an Oxystearinsäure- oder Ricinolsäuretriglycerid), an Glyzerin-mono- und/oder -distearat oder an Sorbitanmono- und/oder -distearat.

Geeignete Tenside für erfindungsgemäße Mittel sind des weiteren z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂-C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆)-estern, Acylsarcosinen, Acyltauriden und Acylisothionaten mit jeweils 8-18 C-Atomen in der Acylgruppe. Auch zwitterionische, ampholytische und nichtionische Tenside sind geeignet, z. B. Oxethylate von Fettsäuremono- und -diglyceriden, von Fettsäure-Sorbitanestern und Alkyl(oligo)-Glucoside.

Die Mund- und Zahnpflegemittel, insbesondere die Zahnpasten, können auch die Unempfindlichkeit der Zähne steigernde Substanzen enthalten, beispielsweise Kaliumsalze wie z. B. Kaliumnitrat, Kaliumcitrat, Kaliumchlorid, Kaliumbicarbonat und Kaliumoxalat. Diese Substanzen sind in den erfindungsgemäßen Mitteln vorzugsweise in Mengen von 0,5 bis 20 Gew.%, besonders bevorzugt von 1,0 bis 15 Gew.%, weiter bevorzugt von 2,5 bis 10 Gew.-% und insbesondere von 4,0 bis 8,0 Gew.%, jeweils bezogen auf das gesamte Mittel, enthalten.

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab. Eine besonders wirksame remineralisierende Substanz stellt das Calcium-glycerophosphat dar, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt.

Die erfindungsgemäßen Zahn- und/oder Mundpflegeprodukte können durch Zugabe von Aromaölen und Süßungsmitteln in ihren organoleptischen Eigenschaften verbessert werden. Als Aromaöle kommen alle für Mund-, Zahn- und/oder Zahnprothesenpflegemittel gebräuchlichen natürlichen und synthetischen Aromen in Frage. Natürliche Aromen können sowohl in Form der aus den Drogen isolierten etherischen Öle als auch der aus diesen isolierten Einzelkomponenten verwendet werden. Bevorzugt sollte wenigstens ein Aromaöl aus der Gruppe Pfefferminzöl, Krauseminzöl, Anisöl, Kümmelöl, Eukalyptusöl, Fenchelöl, Zimtöl, Geraniumöl, Salbeiöl, Thymianöl, Majoranöl, Basilikumöl, Citrusöl, Gaultheriaöl oder eine oder mehrere daraus isolierte synthetisch erzeugten Komponenten dieser Öle enthalten sein. Die wichtigsten Komponenten der genannten Öle sind z.B. Menthol, Carvon, Anethol, Cineol, Eugenol, Zimtaldehyd, Geraniol, Citronellol, Linalool, Salven, Thymol, Terpinen, Terpinol, Methylchavicol und Methylsalicylat. Weitere geeignete Aromen sind z.B. Menthylacetat, Vanillin, Jonone, Linalylacetat, Rhodinol und Piperiton. Als Süßungsmittel eignen sich entweder natürliche Zucker wie Sucrose, Maltose, Lactose und Fructose oder synthetische Süßstoffe wie z.B. Saccharin-Natriumsalz, Natriumcyclamat oder Aspartam.

Weitere übliche Hilfs- und Zusatzstoffe für Zahnpasten sind
- Lösungsmittel und Lösungsvermittler, z.B. niedere einwertige oder mehrwertige Alkohole oder Ether, z.B. Ethanol, 1,2-Propylenglycol, Diethylenglycol oder Butyldiglycol
- Pigmente, wie z.B. Titandioxid
- Farbstoffe
- pH-Stellmittel und Puffersubstanzen wie z.B. Natriumbicarbonat, Natriumcitrat, Natriumbenzoat, Zitronensäure, Phosphorsäure oder saure Salze, z.B. NaH₂PO₄,
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Eine weitere wichtige Gruppe von Inhaltstoffen, die in den erfindungsgemäßen Mitteln enthalten sein kann, sind die sogenannten bioaktiven Gläser.

Der Begriff "bioaktive Gläser" umfaßt im Rahmen der vorliegenden Anmeldung Gläser, welche biologisch wirksam und/oder biologisch aktiv sind. Die biologische Wirksamkeit eines Glases kann sich beispielsweise in dessen antimikrobiellen Eigenschaften zeigen, biologisch aktives Glas unterscheidet sich von herkömmlichen Kalk-Natrium-Silicat-Gläsern dadurch, daß es lebendes Gewebe bindet. Biologisch aktives Glas bezeichnet dabei beispielsweise ein Glas, das eine feste Bindung mit Körpergewebe eingeht, wobei eine Hydroxyl-Apatitschicht ausgebildet wird. Unter bioaktivem Glas wird auch ein Glas verstanden, das antimikrobielle und/oder entzündungshemmende Wirkung zeigt. Die Glaspulver zeigen gegenüber Bakterien, Pilzen sowie Viren eine biozide bzw. eine biostatische Wirkung; sind im Kontakt mit dem Menschen hautverträglich, toxikologisch unbedenklich und insbesondere auch zum Verzehr geeignet.

Erfindungsgemäß besonders bevorzugte Mund- und Zahnpflege- und - reinigungsmittel sind dadurch gekennzeichnet, daß sie 0,2 bis 20 Gew.-%, vorzugsweise 0,4 bis 14 Gew.-%, besonders bevorzugt 0,5 bis 3 Gew.-% und insbesondere 0,6 bis 2 Gew.-% mindestens eines bioaktiven Glases enthalten.

Die erfindungsgemäßen Mund- und Zahnpflege- und -reinigungsmittel dieser Ausführungsform enthalten bioaktives Glas oder Glaspulver oder Glaskeramikpulver oder Kompositmaterialien, welche ein solches bioaktives Glas umfassen. Unter Glaspulvern werden im Rahmen der vorliegenden Anmeldung auch Granulate und Glaskügelchen verstanden.

Aufgrund der Anforderungen an die toxikologische Unbedenklichkeit des Glases sowie deren Eignung zum Verzehr soll das Glaspulver besonders rein sein. Die Belastung durch Schwermetalle ist vorzugsweise gering. So beträgt die Maximalkonzentration im Bereich der kosmetischen Formulierungen vorzugsweise für Pb < 20 ppm, Cd < 5 ppm, As < 5 ppm, Sb < 10 ppm, Hg < 1 ppm, Ni < 10 ppm.

Das unkeramisierte Ausgangsglas, das direkt in den bevorzugten erfindungsgemäßen Zusammensetzungen enthalten oder gegebenenfalls für die Herstellung einer erfindungsgemäß einsetzbaren Glaskeramik verwandt wird, enthält SiO₂ als Netzwerkbildner, vorzugsweise zwischen 35-80 Gew.-%. Bei niedrigeren Konzentrationen nimmt die spontane Kristallisationsneigung stark zu und die chemische Beständigkeit stark ab. Bei höheren SiO₂-Werten kann die Kristallisationsstabilität abnehmen, und die Verarbeitungstemperatur wird deutlich erhöht, so daß sich die Heißformgebungseigenschaften verschlechtern. Na₂O wird als Flußmittel beim Schmelzen des Glases eingesetzt. Bei Konzentrationen kleiner 5% wird das Schmelzverhalten negativ beeinflußt. Natrium ist Bestandteil der sich bei der Keramisierung bildenden Phasen und muß, sofern hohe kristalline Phasenanteile durch die Keramisierung eingestellt werden sollen, in entsprechend hohen Konzentrationen im Glas enthalten sein. K₂O wirkt als Flußmittel beim Schmelzen des Glases. Außerdem wird Kalium in wässrigen Systemen abgegeben. Liegen hohe Kaliumkonzentrationen im Glas vor, werden kaliumhaltige Phasen wie Kalzium-Silicaten ebenfalls ausgeschieden. Über den P₂0₅-Gehalt kann bei silikatischen Gläsern, Glaskeramiken oder Kompositen die chemische Beständigkeit des Glases und damit die lonenabgabe in wässrigen Medien eingestellt werden. Bei Phospahtgläsern ist P₂O₅ Netzwerkbilder. Der P₂O₅-Gehalt liegt vorzugsweise zwischen 0 und 80 Gew.-%. Um die Schmelzbarkeit zu verbessern, kann das Glas bis zu 25 Gew.- % B₂O₃ enthalten. Al₂O₃ wird genutzt, um die chemische Beständigkeit des Glases einzustellen.

Zur Verstärkung der antimikrobiellen, insbesondere der antibakteriellen Eigenschaften der Glaskeramik können antimikrobiell wirkende lonen wie z. B. Ag, Au, I, Ce, Cu, Zn in Konzentrationen kleiner 5 Gew.-% enthalten sein.

Farbgebende Ionen wie z. B. Mn, Cu, Fe, Cr, Co, V, können einzeln oder kombiniert, vorzugsweise in einer Gesamtkonzentration kleiner 1 Gew.-%, enthalten sein.

Üblicherweise wird das Glas bzw. die Glaskeramik in Pulverform eingesetzt. Die Keramisierung kann entweder mit einem Glasblock bzw. Glasribbons erfolgen oder aber mit Glaspulver. Nach der Keramisierung müssen die Glaskeramikblöcke oder Ribbons zu Pulver gemahlen werden. Wurde das Pulver keramisiert, muß gegebenenfalls auch erneut gemahlen werden, um Agglomerate, die während des Keramisierungsschrittes entständen sind, zu entfernen. Die Mahlungen können sowohl trocken als auch in wässrigen oder nicht wässrigen Mahlmedien durchgeführt werden. Üblicherweise liegen die Partikelgrößen kleiner 500 µm. Als zweckmäßig haben sich Partikelgrößen < 100 µm bzw. < 20 µm erwiesen. Besonders geeignet sind Partikelgrößen < 10 µm sowie kleiner 5 µm sowie kleiner 2 µm, siehe weiter unten.

Die in den bevorzugten erfindungsgemäßen Zusammensetzungen enthaltenen bioaktiven Gläser bzw. Glaspulver oder Glaskeramikpulver oder Komposit-Zusammensetzungen umfassen Gläser, die bevorzugt nachfolgende Komponenten umfassen: SiO₂: 35-80 Gew.-%, Na₂O: 0-35 Gew.- %, P₂O₅: 0-80 Gew.-%, MgO: 0-5 Gew.-%, Ag₂O: 0-0,5 Gew.-%, AgJ: 0-0,5 Gew.- %, NaJ: 0-5 Gew.-%, TiO₂: 0-5 Gew.-%, K₂O: 0-35 Gew.-%, ZnO: 0-10 Gew.-%, Al₂O₃: 0-25 Gew.-% undB₂O₃: 0-25 Gew.-% .

Weiterhin können dem Grundglas gemäß obiger Zusammensetzung zur Erzielung weiterer Effekte wie beispielsweise Farbigkeit oder UV-Filterung Ionen wie Fe, Co, Cr, V, Ce, Cu, Mn, Ni, Bi, Sn, Ag, Au, J einzeln oder in Summe bis zu 10 Gew.-% zugegeben werden. Eine weiter Glaszusammensetzung kann wie folgt sein: SiO₂: 35-80 Gew.-%, Na₂O: 0-35 Gew.-%, P₂O₅: 0-80 Gew.-%, MgO: 0-5 Gew.- %, Ag₂O: 0-0,5 Gew.-%, AgJ: 0-0,5 Gew.-%, NaJ: 0-5 Gew.-%, TiO₂: 0-5 Gew.-%, K₂O: 0-35 Gew.-%, ZnO: 0-10 Gew.-%, Al₂O₃: 0-25 Gew.-%, B₂O₃: 0-25 Gew.-%, SnO: 0-5 Gew.-%, CeO₂: 0-3 Gew.- % und Au: 0,001-0,1 Gew.-%.

Besonders bevorzugte erfindungsgemäße Mund- und Zahnpflege- und - reinigungsmittel sind dadurch gekennzeichnet, daß das bioaktive Glas - bezogen auf sein Gewicht - folgende Zusammensetzung aufweist:

| | | |
|---|---|---|
| SiO₂ | 35 bis 60 Gew.-%, vorzugsweise | 40 bis 60 Gew.-%, |
| Na₂O | 0 bis 35 Gew.-%, vorzugsweise | 5 bis 30 Gew.-%, |
| K₂O | 0 bis 35 Gew.-%, vorzugsweise | 0 bis 20 Gew.-%, |
| P₂O₅ | 0 bis 10 Gew.-%, vorzugsweise | 2 bis 10 Gew.-%, |
| MgO | 0 bis 10 Gew.-%, vorzugsweise | 0 bis 5 Gew.-%, |
| CaO | 0 bis 35 Gew.-%, vorzugsweise | 5 bis 30 Gew.-%, |
| Al₂O₃ | 0 bis 25 Gew.-%, vorzugsweise | 0 bis 5 Gew.-%, |
| B₂O₃ | 0 bis 25 Gew.-%, vorzugsweise | 0 bis 5 Gew.-%, |
| TiO₂ | 0 bis 10 Gew.-%, vorzugsweise | 0,1 bis 5 Gew.-%. |

Wie bereits weiter oben erwähnt, wird das bioaktive Glas vorzugsweise in partikulärer Form eingesetzt. Hier sind besonders bevorzugte erfindungsgemäße Mund- und Zahnpflege- und -reinigungsmittel dadurch gekennzeichnet, daß das antimikrobielle Glas Teilchengrößen < 10 µm, vorzugsweise von 0,5 bis 4 µm, besonders bevorzugt von 1 bis 2 µm, aufweist.

Es hat sich gezeigt, daß die enzymhaltigen erfindungsgemäßen Mund- und Zahnpflege- und -reinigungsmittel in ihrer Leistung weiter gesteigert werden können, wenn die Mittel salivationsfördernde Substanzen enthalten.

Unter Salivation versteht man die Speichelproduktion und -freisetzung, im weiteren Sinne auch in unphysiologisch erhöhter Menge. Substanzen, die den Speichelfluß anregen und die Speichelmenge und/oder -freisetzung erhöhen, können aus den unterschiedlichsten Stoffklassen stammen.

Eine erfindungsgemäß beispielsweise geeignete Substanz ist das Pilocarpin, das in den erfindungsgemäßen Mund- und Zahnpflege- und -reinigungsmitteln enthalten sein kann.

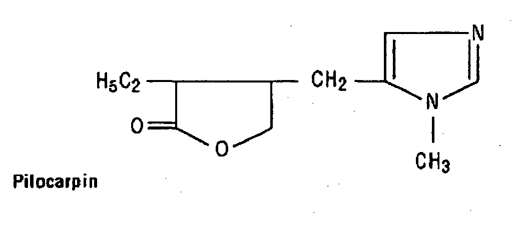

Weitere salivationsfördernde Substanzen sind insbesondere sogenannte Scharfstoffe, d.h. scharf schmeckende und/oder ein Gefühl von Wärme erzeugende Substanzen. Erfindungsgemäß bevorzugte Mund- und Zahnpflege- und -reinigungsmittel sind dadurch gekennzeichnet, daß sie als salivationsfördernde Substanz mindestens eine scharf schmeckende und/oder ein Gefühl von Wärme erzeugende Substanz enthalten.

Als salivationsfördernden Inhaltsstoff enthalten die erfindungsgemäßen Erzeugnisse dieser Ausführungsform eine scharf schmeckende und/oder ein Gefühl von Wärme erzeugende Substanz. Diese Substanzen vermitteln dem Anwender einen scharfen, kribbelnden, mundwässernden oder wärmeerzeugenden Effekt, d.h. sie rufen sensorisch einen Wärmeeindruck oder ein Brennen, oder ein Prickeln, Perlen, Kitzeln oder Sprudeln hervor und fördern dadurch den Speichelfluß.

Erfindungsgemäß bevorzugte Erzeugnisse dieser Ausführungsform enthalten die scharf schmeckende(n) und/oder ein Gefühl von Wärme erzeugende(n) Substanz(en) in Mengen von 0,00001 bis 5 Gew.-%, vorzugsweise von 0,0005 bis 2,5 Gew.-%, weiter bevorzugt von 0,001 bis 1 Gew.-%, besonders bevorzugt von 0,005 bis 0,75 Gew.-% und insbesondere von 0,01 bis 0,5 Gew.-%, jeweils bezogen auf das Gewichts des gesamten Mittels.

Als scharf schmeckende oder ein Gefühl von Wärme erzeugende Substanz kann eine Reihe von Stoffen eingesetzt werden. Bevorzugt sind insbesondere N-Alkylsubstituierte Amide von ungesättigten Carbonsäuren, beispielsweise
- 2E,4E-Decadiensäure-N-Methylamid
- 2E,4E-Decadiensäure-N-Ethylamid
- 2E,4E-Decadiensäure-N-n-Propylamid
- 2E,4E-Decadiensäure-N-Isopropylamid
- 2E,4E-Decadiensäure-N-n-Butylamid
- 2E,4E-Decadiensäure-N-(1-Methylpropyl)-amid
- 2E,4E-Decadiensäure-N-Isobutylamid
- 2E,4E-Decadiensäure-N-tert-Butylamid

- 2E,4Z-Decadiensäure-N-Methylamid
- 2E,4Z-Decadiensäure-N-Ethylamid
- 2E,4Z-Decadiensäure-N-n-Propylamid
- 2E,4Z-Decadiensäure-N-Isopropylamid
- 2E,4Z-Decadiensäure-N-n-Butylamid
- 2E,4Z-Decadiensäure-N-(1-Methylpropyl)-amid
- 2E,4Z-Decadiensäure-N-Isobutylamid
- 2E,4Z-Decadiensäure-N-tert-Butylamid

- 2E,4E,8Z-Decatriensäure-N-Methylamid
- 2E,4E,8Z-Decatriensäure-N-Ethylamid
- 2E,4E,8Z-Decatriensäure-N-n-Propylamid
- 2E,4E,8Z-Decatriensäure-N-Isopropylamid
- 2E,4E,8Z-Decatriensäure-N-n-Butylamid
- 2E,4E,8Z-Decatriensäure-N-(1-Methylpropyl)-amid
- 2E,4E,8Z-Decatriensäure-N-Isobutylamid
- 2E,4E,8Z-Decatriensäure-N-tert-Butylamid

- 2E,4Z,8Z-Decatriensäure-N-Methylamid
- 2E,4Z,8Z-Decatriensäure-N-Ethylamid
- 2E,4Z,8Z-Decatriensäure-N-n-Propylamid
- 2E,4Z,8Z-Decatriensäure-N-Isopropylamid
- 2E,4Z,8Z-Decatriensäure-N-n-Butylamid
- 2E,4Z,8Z-Decatriensäure-N-(1-Methylpropyl)-amid
- 2E,4Z,8Z-Decatriensäure-N-Isobutylamid
- 2E,4Z,8Z-Decatriensäure-N-tert-Butylamid

- 2E,4E,8E-Decatriensäure-N-Methylamid
- 2E,4E,8E-Decatriensäure-N-Ethylamid
- 2E,4E,8E-Decatriensäure-N-n-Propylamid
- 2E,4E,8E-Decatriensäure-N-Isopropylamid
- 2E,4E,8E-Decatriensäure-N-n-Butylamid
- 2E,4E,8E-Decatriensäure-N-(1-Methylpropyl)-amid
- 2E,4E,8E-Decatriensäure-N-Isobutylamid
- 2E,4E,8E-Decatriensäure-N-tert-Butylamid

- 2E,4Z,8E-Decatriensäure-N-Methylamid
- 2E,4Z,8E-Decatriensäure-N-Ethylamid
- 2E,4Z,8E-Decatriensäure-N-n-Propylamid
- 2E,4Z,8E-Decatriensäure-N-Isopropylamid
- 2E,4Z,8E-Decatriensäure-N-n-Butylamid
- 2E,4Z,8E-Decatriensäure-N-(1-Methylpropyl)-amid
- 2E,4Z,8E-Decatriensäure-N-Isobutylamid
- 2E,4Z,8E-Decatriensäure-N-tert-Butylamid

- 2E,6Z,8E-Decatriensäure-N-Methylamid
- 2E,6Z,8E-Decatriensäure-N-Ethylamid
- 2E,6Z,8E-Decatriensäure-N-n-Propylamid
- 2E,6Z,8E-Decatriensäure-N-Isopropylamid
- 2E,6Z,8E-Decatriensäure-N-n-Butylamid
- 2E,6Z,8E-Decatriensäure-N-(1-Methylpropyl)-amid
- 2E,6Z,8E-Decatriensäure-N-Isobutylamid
- 2E,6Z,8E-Decatriensäure-N-tert-Butylamid

- 2E,6E,8E-Decatriensäure-N-Methylamid
- 2E,6E,8E-Decatriensäure-N-Ethylamid
- 2E,6E,8E-Decatriensäure-N-n-Propylamid
- 2E,6E,8E-Decatriensäure-N-Isopropylamid
- 2E,6E,8E-Decatriensäure-N-n-Butylamid
- 2E,6E,8E-Decatriensäure-N-(1-Methylpropyl)-amid
- 2E,6E,8E-Decatriensäure-N-Isobutylamid
- 2E,6E,8E-Decatriensäure-N-tert-Butylamid

- 2E,6Z,8Z-Decatriensäure-N-Methylamid
- 2E,6Z,8Z-Decatriensäure-N-Ethylamid
- 2E,6Z,8Z-Decatriensäure-N-n-Propylamid
- 2E,6Z,8Z-Decatriensäure-N-Isopropylamid
- 2E,6Z,8Z-Decatriensäure-N-n-Butylamid
- 2E,6Z,8Z-Decatriensäure-N-(1-Methylpropyl)-amid
- 2E,6Z,8Z-Decatriensäure-N-Isobutylamid
- 2E,6Z,8Z-Decatriensäure-N-tert-Butylamid

- 2E,6E,8Z-Decatriensäure-N-Methylamid
- 2E,6E,8Z-Decatriensäure-N-Ethylamid
- 2E,6E,8Z-Decatriensäure-N-n-Propylamid
- 2E,6E,8Z-Decatriensäure-N-Isopropylamid
- 2E,6E,8Z-Decatriensäure-N-n-Butylamid
- 2E,6E,8Z-Decatriensäure-N-(1-Methylpropyl)-amid
- 2E,6E,8Z-Decatriensäure-N-Isobutylamid
- 2E,6E,8Z-Decatriensäure-N-tert-Butylamid

- 2E,7Z,9E-Undecatriensäure -N-Methylamid
- 2E,7Z,9E-Undecatriensäure -N-Ethylamid
- 2E,7Z,9E-Undecatriensäure -N-n-Propylamid
- 2E,7Z,9E-Undecatriensäure -N-Isopropylamid
- 2E,7Z,9E-Undecatriensäure -N-n-Butylamid
- 2E,7Z,9E-Undecatriensäure -N-(1-Methylpropyl)-amid
- 2E,7Z,9E-Undecatriensäure-N-isobutylamid
- 2E,7Z,9E-Undecatriensäure -N-tert-Butylamid

- 2E,7E,9E-Undecatriensäure -N-Methylamid
- 2E,7E,9E-Undecatriensäure -N-Ethylamid
- 2E,7E,9E-Undecatriensäure -N-n-Propylamid
- 2E,7E,9E-Undecatriensäure -N-Isopropylamid
- 2E,7E,9E-Undecatriensäure -N-n-Butylamid
- 2E,7E,9E-Undecatriensäure -N-(1-Methylpropyl)-amid
- 2E,7E,9E-Undecatriensäure-N-isobutylamid
- 2E,7E,9E-Undecatriensäure -N-tert-Butylamid

- 2E,7Z,9Z-Undecatriensäure -N-Methylamid
- 2E,7Z,9Z-Undecatriensäure -N-Ethylamid
- 2E,7Z,9Z-Undecatriensäure -N-n-Propylamid
- 2E,7Z,9Z-Undecatriensäure -N-Isopropylamid
- 2E,7Z,9Z-Undecatriensäure -N-n-Butylamid
- 2E,7Z,9Z-Undecatriensäure -N-(1-Methylpropyl)-amid
- 2E,7Z,9Z-Undecatriensäure-N-isobutylamid
- 2E,7Z,9Z-Undecatriensäure -N-tert-Butylamid

- 2E,7Z,9E-Undecatriensäure -N-Methylamid
- 2E,7Z,9E-Undecatriensäure -N-Ethylamid
- 2E,7Z,9E-Undecatriensäure -N-n-Propylamid
- 2E,7Z,9E-Undecatriensäure -N-Isopropylamid
- 2E,7Z,9E-Undecatriensäure -N-n-Butylamid
- 2E,7Z,9E-Undecatriensäure -N-(1-Methylpropyl)-amid
- 2E,7Z,9E-Undecatriensäure-N-isobutylamid
- 2E,7Z,9E-Undecatriensäure -N-tert-Butylamid

Selbstverständlich sind auch andere Substitutionsmuster am Stickstoffatom möglich und bevorzugt, beispielsweise längerkettige n-Alkylreste (...-N-n-Pentylamid, ...-N-n-Pentylamid, ...-N-n-Pentylamid, ...-N-n-Pentylamid, ...-N-n-Pentylamid, ...-N-n-Hexylamid, ...-N-n-Heptylamid, ...-N-n-Octylamid, ...-N-n-Nonylamid, ...-N-n-Decylamid, ...-N-n-Undecylamid, ...-N-n-Dodecylamid, ...-N-n-Tridecylamid, usw.) oder disubstituierte ...-N,N-Dialkylamide wie ...-N,N-dimethylamid, ...-N,N-diethylamid, ...-N,N-di-n-propylamid, ...-N,N-diisopropylamid, ...-N,N-di-n-butylamid, ...-N,N-di(1-Methylpropyl)amid, ...-N,N-düsobutylamid, ...-N,N-di-tert-butylamid, ...-N,N-methyl-ethylamid, ...-N,N-methyl-n-propylamid, ...-N,N-methyl-isopropylamid, ...-N,N-ethyl-n-propylamid, ...-N,N-ethyl-isopropylamid, usw..

Unter den genannten Verbindungen sind einige im Rahmen der vorliegenden Erfindung besonders bevorzugt. Diese sind nachfolgend aufgeführt:
2E,6Z,8E-Decatriensäure-N-isobutylamid (N-Isobutyl-2E,6Z,8E-decatrienamid, auch Spilanthol oder Affinin genannt): 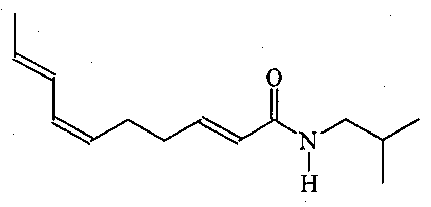
2E,4E,8Z-Decatriensäure-N-isobutylamid (N-Isobutyl-2E,4E,8Z-decatrienamid, auch Isoaffinin genannt): 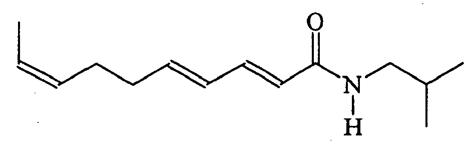
2E,7Z,9E-Undecatriensäure-N-isobutylamid (N-Isobutyl-2E,7Z,9E-undecatrienamid): 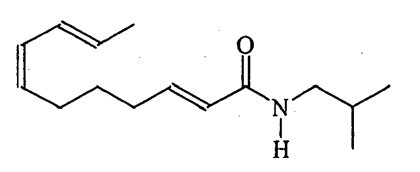
2E,4Z-Decadiensäure-N-isobutylamid (cis-Pellitorin): 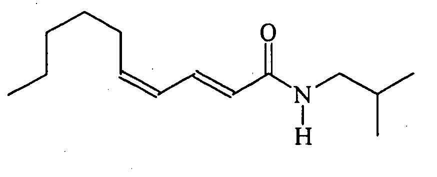
2E,4E-Decadiensäure-N-isobutylamid (trans-Pellitorin):

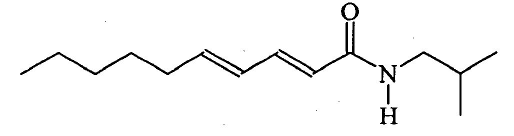

### Ferulasäureamide, beispielsweise

Ferulasäure-N-Vanillylamid: 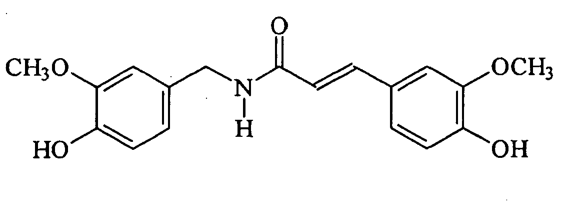
*N*-[2-(4-Hydroxy-3-methoxyphenyl)ethyl]-3-(4-hydroxy-3-methoxy-phenyl)-(2*E*)-propensäureamid (trans-Feruloylmethoxytyramin): 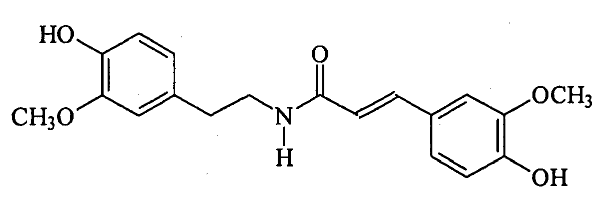
*N*-[2-(4-Hydroxy-3-methoxyphenyl)ethyl]-3-(4-hydroxy-3-methoxy-phenyl)-(2*Z*)-propensäureamid (cis-Feruloylmethoxytyramin): 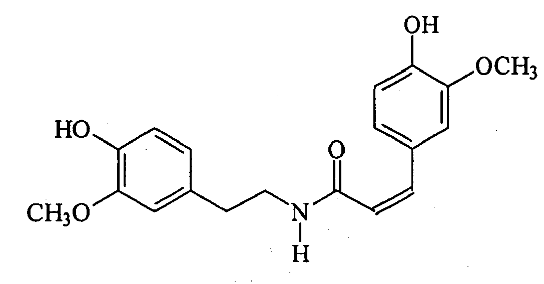
*N*-[2-(4-Hydroxy-3-methoxyphenyl)ethyl]-3-(4-hydroxy-3-methoxy-phenyl)-propansäureamid (Dihydroferuloylmethoxytyramin): 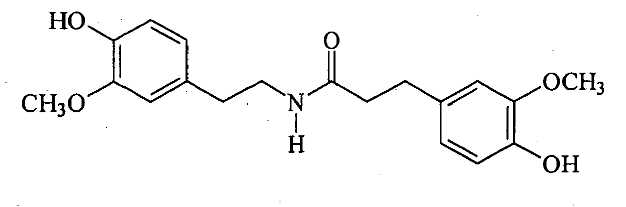
*N*-[2-(3,4- Dihydroxyphenyl)ethyl]-3-(4-hydroxy-3-methoxy-phenyl)-(2*E*)-propensäureamid (trans-Feruloyldopamin): 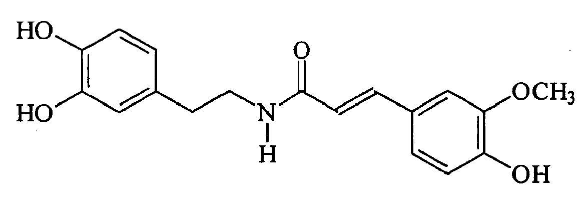
*N*-[2-(3,4-Dihydroxyphenyl) ethyl]-3-(4-hydroxy-3-methoxy-phenyl)-(2*Z*)-propensäureamid (cis- Feruloyldopamin): 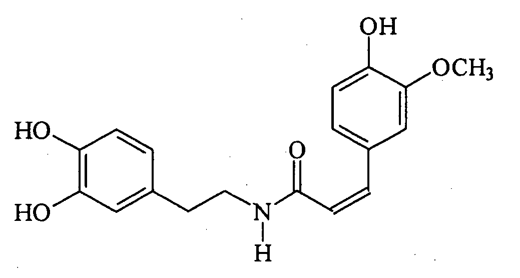
*N*-[2-(4-Hydroxyphenyl)ethyl]-3-(3,4-dihydroxyphenyl)-(2*E*)-propensäureamid (trans-Caffeoyltyramin): 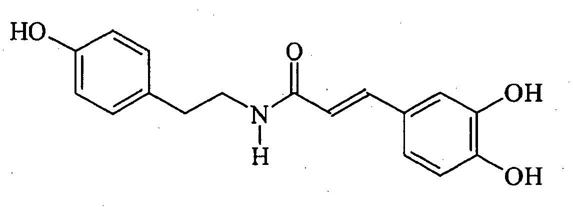
*N*-[2-(4-Hydroxyphenyl)ethyl]-3-(3,4-dihydroxyphenyl)-(2Z)- propensäureamid (cis-Caffeoyltyramin): 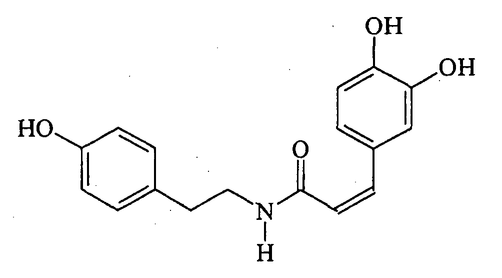
*N*-[2-(3,4-Dimethoxyphenyl)ethyl]-3-(3,4-dimethophenyl)-(2*E*)-propensäureamid (trans- Rubenamin): 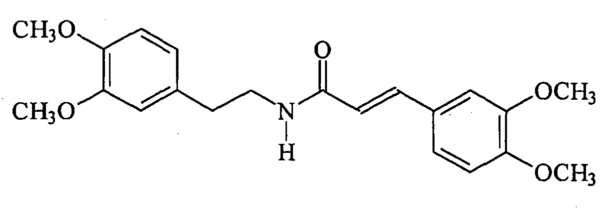
*N*-[2-(3,4-dimethoxyphenyl)ethyl]-3-(3,4- dimethoxy-phenyl)-(2*Z*)-propensäureamid (cis-Rubenamin):

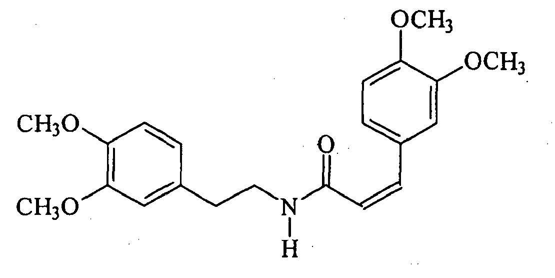

Weitere im Rahmen der vorliegenden Erfindung mit besonderem Vorzug einsetzbare Scharfstoffe sind beispielsweise Extrakte aus Naturpflanzen. Scharf schmeckende pflanzliche Extrakte können alle physiologisch unbedenklichen pflanzlichen Extrakte sein, die einen scharfen oder warmen sensorischen Eindruck hervorrufen. Bevorzugt als scharf schmeckende pflanzliche Extrakte sind beispielsweise Pfefferextrakt (*Piper ssp.,* insbesondere *Piper nigrum*), Wasserpfefferextrakt (*Polygonum ssp.* ,insbesondere *Polygonum hydropiper*), Extrakte aus *Allium* ssp.(insbesondere Zwiebel und Knoblauchextrakte), Extrakte aus Rettich (*Raphanus ssp*.), Meerrettichextrakte (*Cochlearia armoracia*), Extrakte aus schwarzem (*Brassica nigra*), wildem oder gelbem Senf (*Sinapis ssp.,* insbesondere *Sinapis arvensis* und *Sinapis alba*), Bertramwurzel-Extrakte (*Anacyclus ssp.,* insbesondere *Anacyclus pyrethrum*L.), Sonnenhutextrakte ( *Echinaceae ssp*.), Extrakten aus Szechuan-Pfeffer (*Zanthoxylum ssp.,* insbesondere *Zanthoxylum piperitum*), Spilanthesextrakt (*Spilanthes ssp.,* insbesondere *Spilanthes acmella*), Chiliextrakt (*Capsicum ssp.,* insbesondere *Capsicum frutescens* ), Paradieskörner-Extrakt ( *Aframomum ssp.*, insbesondere *Aframomum melegueta*[Rose] K. Schum.),Ingwerextrakt (*Zingiber* ssp.,insbesondere *Zingiber officinale*) und Galangaextrakt (*Kaempferia galanga* oder *Alpinia galanga*).

Eine besonders geeignete Substanz ist das aus dem Ingwerextrakt stammende Gingerol:

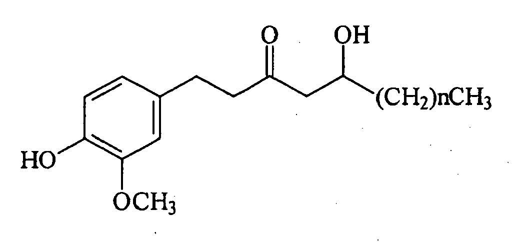

Einsetzbar ist auch N-Ethyl-p-menthan-3-carboxamid (N-Ethyl-5-Methyl-2-isopropylcyclohexancarboxamid):

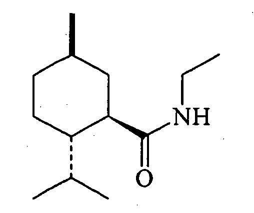

Andere scharf schmeckende oder ein Gefühl von Wärme erzeugende Substanzen können z.B. sein Capsaicin, Dihydrocapsaicin, Gingerol, Paradol, Shogaol, Piperin, Carbonsäure-N- vanillylamide, insbesondere Nonansäure-N-vanillylamid, 2-Alkensäureamide, insbesondere 2-Nonensäure-N-isobutylamid, 2-Nonensäure-N-4-hydroxy-3- methoxyphenylamid, Alkylether von 4-Hydroxy-3-methoxybenzylalkohol, insbesonders 4-Hydroxy-3-methoxybenzyl-n-butylether, Alkylether von 3- Hydroxy-4-methoxybenzylalkohol, Alkylether von 3,4-Dimethoxybenzylalkohol, Alkylether von 3-Ethoxy-4-hydroxybenzylalkohol, Alkylether von 3,4-Methylendioxybenzylalkohol, Nicotinaldehyd, Methylnicotinat, Propylnicotinat, 2-Butoxyethylnicotinat, Benzylnicotinat, 1-Acetoxychavicol, Polygodial oder Isodrimeninol.

Bevorzugte erfindungsgemäße remineralisierende Erzeugnisse sind dadurch gekennzeichnet, daß sie mindestens einen Scharfstoff aus der Gruppe der N-Alkyl-substituierte Amide von ungesättigten Carbonsäuren, vorzugsweise
a. 2E,6Z,8E-Decatriensäure-N-isobutylamid (Spilanthol) und/oder
b. 2E,4E,8Z-Decatriensäure-N-isobutylamid und/oder
c. 2E,7Z,9E-Undecatriensäure-N-isobutylamid und/oder
d. 2E,4Z-Decadiensäure-N-isobutylamid (cis-Pellitorin) und/oder
e. 2E,4E-Decadiensäure-N-isobutylamid (trans-Pellitorin) und/oder
f. Ferulasäure-N-Vanillylamid und/oder
g. *N*-[2-(4-Hydroxy-3-methoxyphenyl)ethyl]-3-(4-hydroxy-3-methoxyphenyl)- (2*E*)-propensäureamid (trans-Feruloylmethoxytyramin) und/oder
h. *N*-[2- (4-Hydroxy-3-methoxyphenyl)ethyl]-3-(4-hydroxy-3-methoxyphenyl)-(2*Z*)-propensäureamid (cis-Feruloylmethoxytyramin) und/oder
i. *N*-[2-(4- Hydroxy-3-methoxyphenyl)ethyl]-3-(4-hydroxy-3-methoxyphenyl)- propansäureamid (Dihydroferuloylmethoxytyramin) und/oder
j. *N*-[2-(3,4- Dihydroxyphenyl)ethyl]-3-(4-hydroxy-3-methoxy-phenyl)-(2*E*)- propensäureamid (trans-Feruloyldopamin) und/oder
k. *N*-[2-(3,4-Dihydroxyphenyl) ethyl]-3-(4-hydroxy-3-methoxy-phenyl)-(2*Z*)-propensäureamid (cis- Feruloyldopamin) und/oder
l. *N*-[2-(4-Hydroxyphenyl)ethyl]-3-(3,4-dihydroxyphenyl)-(2*E*)-propensäureamid (trans-Caffeoyltyramin) und/oder
m. *N*-[2-(4-Hydroxyphenyl)ethyl]-3-(3,4-dihydroxyphenyl)-(2*Z*)-propensäureamid (cis-Caffeoyltyramin) und/oder
n. *N*-[2-(3,4-Dimethoxyphenyl) ethyl]-3-(3,4-dimethoxyphenyl)-(2*E*)-propensäureamid (trans- Rubenamin) und/oder
o. *N-*[2-(3,4-dimethoxyphenyl)ethyl]-3-(3,4-dimethoxy-phenyl)-(2*Z*)-propensäureamid (cis-Rubenamin)
enthalten.

Zusätzlich zu den genannten Scharfstoffen oder an ihrer Stelle können auch weitere scharf schmeckende und/oder ein Gefühl von Wärme erzeugende Substanzen in die erfindungsgemäßen Erzeugnisse eingearbeitet werden.

Als besonders geeignet haben sich im Rahmen der vorliegenden Erfindung alkylsubstituierte Dioxane der Formel 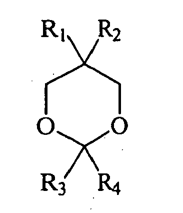 erwiesen, in der R1 und R2 unabhängig voneinander ausgewählt sind aus -H, -CH₃, -CH₂CH₃ und R3 und R4 unabhängig voneinander ausgewählt sind aus -H, -CH₃, -CH₂CH₃, -CH₂CH₂CH₃, -CH(CH₃)₂.

Als weiterhin besonders geeignet haben sich im Rahmen der vorliegenden Erfindung Phenylester der Formel 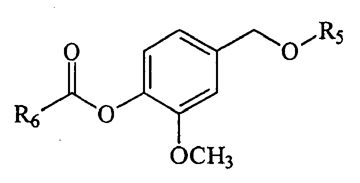 erwiesen, in der R5 für -CH₃ oder einen geradkettigen oder verzweigten Alkyl- oder Alkenylrest mit 2 bis 8 Kohlenstoffatomen und R6 für -CH₃ oder einen geradkettigen oder verzweigten Alkyl- oder Alkenylrest mit 2 bis 8 Kohlenstoffatomen oder eine Alkoxygruppe mit 1 bis 3 Kohlenstoffatomen steht

Als weiterhin besonders geeignet haben sich im Rahmen der vorliegenden Erfindung Carvonacetale der Formel 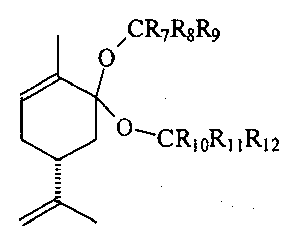 erwiesen, in der R7 bis R12 unabhängig voneinander ausgewählt sind aus -H, -CH₃, -CH₂CH₃, -CH₂CH₂CH₃, -CH(CH₃)₂, -CH₂ CH₂CH₂CH₃, -CH₂CH(CH₃)₂, -CH(CH₃)CH₂CH₃, -C(CH₃)₃ oder R9 und R10 zusammen eine chemische Bindung oder eine Gruppe -(CR13R14)ₓ bedeuten, worin x für die Werte 1 oder 2 steht und R13 und R14 unabhängig voneinander ausgewählt sind aus -H, -CH₃, -CH₂CH₃, -CH₂CH₂CH₃, -CH(CH₃)₂, -CH₂ CH₂CH₂CH₃, -CH₂CH(CH₃)₂, -CH(CH₃)CH₂CH₃, -C(CH₃)₃.

Überraschenderweise wurde ferner gefunden, dass die erfindungsgemäßen Enzyme besonders vorteilhaft in flüssigen bzw. halbflüssigen Zahnputzzubereitungen nutzbar gemacht werden können, insbesondere in durchscheinenden oder transparenten Formulierungen mit hoher Verbraucherakzeptanz.

Die Enzyme können hierbei in einer besonders bevorzugten Ausführungsform in eine spezielle Matrix stabil eingearbeitet werden, wobei die Reinigungsleistungen entsprechender Mittel - die auch transparent formuliert werden können - herkömmlichen Mitteln überlegen ist.

Weiterer bevorzugter Gegenstand der vorliegenden Erfindung ist daher ein Mund- und Zahnpflege- und -reinigungsmittel, enthaltend, jeweils bezogen auf sein Gewicht, neben mindestens einem der zuvor genannten erfindungsgemäßen Enzyme sowie 1-30% Putzkörper 15 bis 35 Gew.-% Wasser sowie 35 bis 55 Gew.-% mindestens eines mehrwertigen Alkohols aus der Gruppe Sorbit und/oder Glycerin und/oder 1,2-Propylenglycol. Daneben können natürlich auch weitere Inhaltsstoffe, wie zuvor angegeben, enthalten sein.

Erfindungsgemäß bevorzugte Mund- und Zahnpflege- und -reinigungsmittel sind in dieser Ausführungsform dadurch gekennzeichnet, dass sie 20 bis 34 Gew.-%, vorzugsweise 22,5 bis 33 Gew.-%, besonders bevorzugt 24 bis 32 Gew.-% und insbesondere 25 bis 31 Gew.-% Wasser enthalten.

In die Berechnung des Gesamt-Wassergehaltes der Mittel fließen die Wasseranteile wässriger Lösungen selbstverständlich mit ein. Wird also beispielsweise Sorbit in Form einer 70 Gew.-%-igen Lösung eingesetzt, so beträgt der Sorbit-Anteil das 0,7-fache des eingesetzten Gewichtsanteils, während der Wasseranteil um das 0,3-fache des eingesetzten Gewichtsanteils erhöht wird. Analog ist auch mit wässrigen Lösungen von Farb- oder Aromastoffen usw. zu verfahren.

Die erfindungsgemäßen Mund- und Zahnpflege- und -reinigungsmittel enthalten in dieser bevorzugten Ausführungsform als weiteren wesentlichen Inhaltsstoff mindestens einen mehrwertigen Alkohol aus der Gruppe Sorbit, Glycerin und 1,2-Propylenglycol in einer Menge von 35 bis 55 Gew.-%.

Hier sind erfindungsgemäße Mund- und Zahnpflege- und -reinigungsmittel bevorzugt, die 37,5 bis 52,5 Gew.-%, vorzugsweise 39 bis 51 Gew.-%, besonders bevorzugt 40 bis 50 Gew.-% und insbesondere 42 bis 49 Gew.-% mindestens eines mehrwertigen Alkohols aus der Gruppe Sorbit und/oder Glycerin und/oder 1,2-Propylenglycol enthalten.

Sorbit (auch als Glucit bezeichnet) ist ein Zuckeralkohol von Glucose, also ein Hexit. Sorbit ist durch Hydrierung von Glucose herstellbar, spaltet intramolekular relativ leicht ein oder zwei Moleküle Wasser ab und bildet cyclische Ether. Sorbit läßt sich durch die Formel 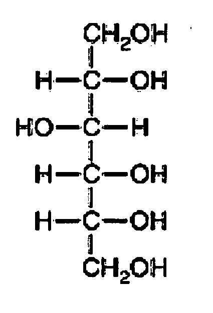 beschreiben und kommt in Form farbloser, mäßig hygroskopischer, optisch aktiver Nadeln, welche sich leicht in Wasser lösen, in den Handel.

Glycerin (1,2,3-Propantriol, 1,2,3-Trihydroxypropan, Glycerol, Ölsüß, INCI-Bezeichnung: Glycerin, E 422) ist eine farblose, klare, schwerbewegliche, geruchlose, süß schmeckende, hygroskopische Flüssigkeit, die mit Wasser und Alkohol in jedem Verhältnis mischbar ist.

### Glycerin läßt sich durch die Formel

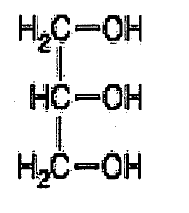

beschreiben. Die Herstellung von Glycerin erfolgte ursprünglich als Nebenprodukt der Fettverseifung. Die heutigen technischen Verfahren gehen von Propen aus, das über die Zwischenstufen Allylchlorid und Epichlorhydrin zu Glycerin verarbeitet wird. Ein weiteres technisches Verfahren ist die Hydroxylierung von Allylalkohol mit Wasserstoffperoxid am WO₃-Kontakt über die Stufe des Glycids.

1,2-Propylenglykol (1,2-Propandiol) ist eine farblose und stark hygroskopische Flüssigkeit, die in jedem Verhältnis mit Wasser und Alkoholen (wie Methanol, Ethanol, Propanolen, Butanolen) mischbar ist. Technisches 1,2-Propandiol ist ein Racemat aus (-)-(*R*)- und (+)-(*S*)-1,2-Prpylenglycol. Die Herstellung erfolgt über direkte Hydrolyse von Propylenoxid. Da 1,2-P. mit Propylenoxid weiterreagiert, entsteht dabei eine Mischung aus 1,2- und Tripropylenglykol, die durch Destillation getrennt werden muß. 1,2-Propylenglycol kann auch aus nachwachsenden Rohstoffen über drei unterschiedliche Routen hergestellt werden: a) Katalytische Hydrierung von Zuckern; b) Gärung von Zuckern zu Milchsäure und anschließend Hydrierung des Milchsäureesters; c) direkte Fermentation von Zuckern.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1) : (0,1-0,5) erwiesen.

Neben Sorbit bzw. Glycerin bzw. 1,2-Propylenglycol, von denen mindestens ein Stoff in den erfindungsgemäßen Mitteln enthalten ist, wobei die Gesamtmenge dieser drei Stoffe mindestens 35 Gew.-% (bezogen auf das Mittel) beträgt, eignen sich als weitere mehrwertige Alkohole solche mit mindestens 2 OH-Gruppen, vorzugsweise Mannit, Xylitol, Polyethylenglycol, Polypropylenglycol und deren Mischungen.

Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden.

Wichtige Vertreter von Polyhydroxyverbindungen mit 2 OH-Gruppen sind auch die Polyethylen- und Polypropylenglycole.

Als bevorzugte weitere mehwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Sorbit, so dass Mittel, die außer Sorbit keine anderen mehrwertigen Alkohole enthalten, besonders bevorzugt sind.

In einer weiteren besonderen Ausführungsform ist das erfindungsgemäße Mittel ein Prothesenreiniger oder ein Prothesenhaftmittel.

Für erfindungsgemäß bevorzugte Prothesenreiniger, insbesondere Prothesenreinigungstabletten und -pulver, eignen sich neben den schon genannten Inhaltsstoffen für die Mund-, Zahn- und/oder Zahnprothesenpflege zusätzlich noch Per-Verbindungen wie beispielsweise Peroxoborat, Peroxomonosulfat oder Percarbonat. Sie haben den Vorteil, dass sie neben der Bleichwirkung gleichzeitig auch desodorierend und/oder desinfizierend wirken. Der Einsatz solcher Per-Verbindungen in Prothesenreinigern beträgt zwischen 0,01 und 10 Gew.-%, insbesondere zwischen 0,5 und 5 Gew.-%.

Der pH-Wert des Prothesenreinigers kann zwischen pH 4 und pH 12, insbesondere zwischen pH 5 und pH 11 liegen.

Für die Prothesenreinigungstabletten sind zusätzlich noch weitere Hilfsstoffe notwendig, wie beispielsweise Mittel, die einen sprudelnden Effekt hervorrufen, wie z.B. CO₂ freisetzende Stoffe wie Natriumhydrogencarbonat, Füllstoffe, z.B. Natriumsulfat oder Dextrose, Gleitmittel, z.B. Magnesiumstearat, Fließregulierungsmittel, wie beispielsweise kolloidales Siliziumdioxid und Granuliermittel, wie die bereits erwähnten hochmolekularen Polyethylenglykole oder Polyvinylpyrrolidon.

Prothesenhaftmittel können als Pulver, Cremes, Folien oder Flüssigkeiten angeboten werden und unterstützen die Haftung der Prothesen. Als Wirkstoffe sind natürliche und synthetische Quellstoffe geeignet. Als natürliche Quellstoffe sind neben Alginaten auch Pflanzengummen, wie z.B. Gummi arabicum, Traganth und Karaya-Gummi sowie natürlicher Kautschuk aufzufassen. Insbesondere haben sich Alginate und synthetische Quellstoffe, wie z.B. Natriumcarboxymethylcellulose, hochmolekulare Ethylenoxid-Copolymere, Salze der Poly(vinyl-ether-co-maleinsäure) und Polyacrylamide, als besonders geeignet herausgestellt.

Als Hilfsstoffe für pastöse und flüssige Produkte eignen sich besonders hydrophobe Grundlagen, insbesondere Kohlenwasserstoffe, wie beispielsweise Weißes Vaselin (DAB) oder Paraffinöl.

### Ausführungsbeispiele:

### a) Rezepturen

Getestet wurden jeweils eine 2in1 Formulierung, eine Pastenrezeptur und eine Gelrezeptur. Je Gramm der Formulierung wurden folgende Enzymkonzentrationen eingesetzt:
β-Glucanase (Firma Biopract GmbH, Organismus: Bacillus amyloliquefaqciens, EC: 3.2.1.6): 1 U (µmol/min)
Protease B7020 (Firma Sternzym; Organismus: Aspergillus oryzae): 0,8 U Neutrale Protease (Firma Lyven; Organismus:Aspergillus oryzae; EC: 3.4.24.28): 1,8 U
Corolase (Firma AB Enzymes; Organismus: Apergillus sojae; EC: 3.4.21.15 und 3.4.21.63): 1,1 U
α-Amylase (Firma Lyven; Organismus Bacillus subtilis; EC: 3.2.1.1): 178 U
Saure Protease (Firma Lyven; Organismus Aspergillus niger; EC3.4.23.18): 31 U

Die Bestimmung der Aktivitäten erfolgte für
die saure Protease mittels der Azocoll-Methode bei pH 4,0,
die anderen Proteasen und die Corolase nach der AAPF- Methode bei pH 7,0,
die α-Amylase nach der DNS-Methode bei pH 7,0,
die β-Glucanase nach der PAHBAH-Methode bei pH 7,0.

Die Enzymaktivitäten wurden bei 25 °C in 100 mM Kalium-Phosphat-Puffer gemessen.

### b) Medien

### Künstlicher Speichel:

0.1 % Lab Lemco Powder, 0.2 % Hefeextrakt, 0.5 % Pepton, 0.25 % Mucin, 6 mM NaCl, 2.7 mM KCI, 3.5 mM KH₂PO₄, 1.5 mM K₂HPO₄, 0.05 % Harnstoff, 1.8 mM CaCl2, 10 µM Hemin, pH 6,65.

### Plaque-Anzucht-Medium:

Künstlicher Speichel/Brain Heart Infusion Medium (Difco) (3/1) + 1 % Glucose

### c) Überprüfung der Plaque-Entfernung

### Plaque-Anzucht:

Eine Mischung der Stämme *F. nucelatum, A. naeslundii, S. gordonii,* und *S. mutans* wurden für 24 h bei 37 °C unter anaeroben Bedingungen im Plaque-Anzucht-Medium in sterilen 48 well Mikrotiterplatten kultiviert. Nach dieser Zeit wurde das Medium von den Oberflächen entfernt, die Plaque verblieb auf den Oberflächen.

### Überprüfung der Plaque-Entfernung:

Enzymhaltige bzw. enzymfreie Produktformulierungen wurden 30 % in Wasser gelöst. Diese verdünnten Produktformulierungen wurden anschließend für 3 Minuten bei Raumtemperatur unter leichten Schüttelbewegungen (600 rpm Titramax 1000) auf die Plaque aufgebracht. Danach wurde die Lösung abgesaugt. Die verbleibende Plaque wurde 2 Mal mit Wasser gespült (beim 1. Mal für 3 Minuten, 600 rpm und Raumtemperatur; beim 2. mal für 1 Minute 600 rpm und Raumtemperatur).
Zur Visualisierung wurde die verbleibende Plaque mit 0,01 % Safranin O oder Comassie Blau Lösung für 15 Minuten gefärbt. Nicht gebundener Farbstoff wurde mit Wasser entfernt.
Zur Ermittlung der Plaquemenge wurde der Farbstoff mit 30 % Essigsäure extrahiert und photometrisch quantifiziert.

### Ergebnisse der Plaqueentfernung:

### (Angaben in % verbleibender Plaque)

| | 2in1 | Paste | Gel |
|---|---|---|---|
| Ohne Enzym | 89,5 | 63,5 | 64,7 |
| β-Glucanase | 1,9 | 14,8 | 3,7 |
| Saure Protease | 73,8 | 73,8 | 32,9 |
| Protease B7020 | 1,2 | 9,0 | 3,5 |
| Neutrale Protease | 0,8 | 8,3 | 3,2 |
| Corolase | 4,7 | 9,5 | 3,9 |
| α-Amylase | 1,5 | 9,5 | 4,8 |

### Beispielrezepturen

Die folgenden Rezepturen sind Beispiele für Zusammensetzungen, die erfindungsgemäße Enzyme enthalten können. Die Mengenangabe der Komponenten erfolgt in Gew.-%, jeweils bezogen auf die Gesamtzusammensetzung.

| Zusammensetzung | 1 | 2 | 3 | 4 | 5 |
|---|---|---|---|---|---|
| Sident 8 | 14,0 | - | - | - | - |
| Sorbosil AC 39 | - | 14,0 | - | - | 14,0 |
| Zeodent 113 | - | - | 14,0 | - | - |
| Zeodent 623 | - | - | - | 14,0 | - |
| Poliertonerde P10 feinst | 1,0 | 1,0 | 1,0 | 1,0 | 0,5 |
| Na5P3010 (Na-Tripolyphoshat) | 5,0 | 5,0 | 5,0 | 5,0 | 5,0 |
| Na2PO3F (Na-Monofluorophosphat) | - | 0,8 | - | - | - |
| NaF | 0,24 | - | 0,24 | 0,24 | 0,24 |
| Na-Saccharinat | 0,1 | 0,1 | 0,1 | 0,1 | 0,1 |
| Titandioxid | 0,5 | 0,5 | 0,5 | 0,5 | 0,5 |
| PHB-Methylester | 0,1 | 0,1 | 0,1 | 0,1 | 0,1 |
| Carboxymethylcellulose | 1,25 | 1,25 | 1,25 | 1,25 | 1,25 |
| Sorbit (70 %ig) | 32,0 | 32,0 | 32,0 | 32,0 | 32,0 |
| 1,2-Propylenglycol | 5,0 | 5,0 | 5,0 | 5,0 | 5,0 |
| Cremophor RH 60 | 1,0 | - | 1,0 | 1,0 | 1,0 |
| Arlatone 289 | - | 1,0 | - | - | - |
| Aromaöl | 0,8 | 0,8 | 0,8 | 0,8 | 0,8 |
| Wasser | ad 100 | ad 100 | ad 100 | ad 100 | ad 100 |

| Zusammensetzung | 6 | 7 | 8 | 9 |
|---|---|---|---|---|
| Sorbosil AC 39 | 14 | 14 | 14 | 14 |
| Poliertonerde P 10 feinst | 1 | - | - | - |
| Precarb 100 | - | 2 | 2 | 2 |
| Na5P3010 | 10 | 10 | 5 | 0 |
| Na2PO3F | 0,8 | 0,8 | 0,8 | 0,8 |
| NaF | - | - | - | - |
| Na-Saccharinat | 0,1 | 0,1 | 0,1 | 0,1 |
| Titandioxid | 0,5 | 0,5 | 0,5 | 0,5 |
| PHB-Methylester | 0,1 | 0,1 | 0,1 | 0,1 |
| Carboxymethylcellulose | 1,25 | 1,25 | 1,25 | 1,25 |
| Sorbit (70 %ig) | 32,0 | 32,0 | 32,0 | 32,0 |
| 1,2-Propylenglycol | 5,0 | 5,0 | 5,0 | 5,0 |
| Arlatone 289 | 1,0 | 1,0 | 1,0 | 1,0 |
| Aromaöl | 0,8 | 0,8 | 0,8 | 0,8 |
| Wasser | ad 100 | ad 100 | ad 100 | ad 100 |

| Zusammensetzung | 10 | 11 | 12 | 13 |
|---|---|---|---|---|
| Sident^{®} 8 | 10,0 | 10,0 | 10,0 | 10,0 |
| Sident^{®} 22S | 7,0 | 7,0 | 7,0 | 7,0 |
| Sipernat^{®} 320DS | 0,8 | 0,8 | 0,8 | 0,8 |
| glycerinische Lösung enthaltend 10 Gew.-% Hydroxylapatit | 1,0 | 0,1 | 1,0 | 0,1 |
| Polywachs^{®} 1550 | 2,0 | 2,0 | 2,0 | 2,0 |
| Texapon^{®} K 1296 | 1,5 | 1,5 | 1,5 | 1,5 |
| Titandioxid | 1,0 | 1,0 | 1,0 | 1,0 |
| Cekol^{®} 500 T | 1,0 | 1,0 | 1,0 | 1,0 |
| Na-Fluorid | 0,33 | 0,33 | 0,33 | 0,33 |
| Na-Benzoat | 0,25 | 0,25 | 0,25 | 0,25 |
| Aroma | 1,0 | 1,0 | 1,0 | 1,0 |
| Tagat^{®} S | 0,2 | - | - | 0,2 |
| Na-Saccharinat | 0,15 | 0,15 | 0,15 | 0,15 |
| Tri-Natriumphosphat | 0,10 | 0,10 | 0,10 | 0,10 |
| Sorbit (70 %ig in Wasser) | 31,0 | 31,0 | 31,0 | 31,0 |
| Wasser | ad 100 | ad 100 | ad 100 | ad 100 |

### 2in1-Formulierungen:

| | 14 | 15 | 16 | 17 | 18 |
|---|---|---|---|---|---|
| Sorbit (70 % DAB) | 60 | 62,5 | 65 | 67,5 | 70,0 |
| Ethanol (96%) | - | - | - | - | 2,0 |
| Fällungskieselsäure : Sident 8 | 10,0 | 10,0 | 10,0 | 10,0 | 12,0 |
| Fällungskieselsäure : Sident 22S | 8,5 | 8,5 | 8,5 | 8,5 | - |
| Poliertonerde P10 feinst | - | 1,0 | - | - | - |
| Dinatriumphosphat, wasserfrei | 0,1 | 0,1 | 0,1 | 0,1 | 0,1 |
| Trinatriumphosphat, wasserfrei | 0,2 | 0,2 | 0,2 | 0,2 | - |
| Na-Monofluorophosphat Na2PO3F | - | - | 1,0 | 1,0 | 1,0 |
| Natriumfluorid | 0,32 | 0,32 | - | - | - |
| Polyethylenglycol (MG : 1500) | 1,5 | 1,5 | 1,5 | 1,5 | 1,5 |
| Titandioxid | 0,5 | 0,5 | 0,5 | 0,5 | - |
| Na-Laurylsulfat | 1,5 | 1,5 | 1,5 | 1,5 | 1,5 |
| Saccharin-Na | 0,1 | 0,1 | 0,1 | 0,1 | 0,1 |
| Xanthan | 0,4 | 0,4 | 0,4 | 0,4 | 0,4 |
| Aroma | 1,2 | 1,2 | 1,2 | 1,2 | 1,2 |
| Wasser | ad 100 | ad 100 | ad 100 | ad 100 | ad 100 |

| | 19 | 20 | 21 | 22 |
|---|---|---|---|---|
| Sident® 8 | 14,0 | 14,0 | 14,0 | 14,0 |
| Sorbosil® AC 33 | 1,0 | 1,0 | - | - |
| Poliertonerde P10 feinst | - | - | 1,0 | 1,0 |
| Glycerin (86%ig DAB) | 18,0 | 18,0 | 18,0 | 18,0 |
| Sorbit (70%ig) | 14,0 | 14,0 | 14,0 | 14,0 |
| 1,2-Propylenglycol | 5,0 | 5,0 | 5,0 | 5,0 |
| AHP 100 | 0,5 | 0,5 | 0,5 | 0,5 |
| NaF | 0,32 | 0,32 | 0,32 | 0,32 |
| Saccharin-Na | 0,2 | 0,2 | 0,2 | 0,2 |
| Na2HPO4 | 1,5 | 1,5 | 1,5 | 1,5 |
| Na3PO4 | 0,5 | 0,5 | 0,5 | 0,5 |
| TiO2 | - | - | 0,5 | 0,5 |
| Timiron® Diamond Cluster MP 149 | 0,2 | 0,2 | - | - |
| Brillant Blue 1%ig (Acid Blue 9) | 0,22 | 0,22 | - | - |
| pHB-Methylester | 0,1 | 0,1 | 0,1 | 0,1 |
| Keltrol® F | 1,25 | 1,25 | 1,25 | 1,25 |
| Texapon® K1296 | 1,5 | 1,5 | 1,5 | 1,5 |
| Tego Betain® BL 215 | 0,6 | 0,6 | 0,6 | 0,6 |
| Triclosan | 0,3 | - | 0,3 | - |
| Jojobaöl | 1,0 | 0,5 | 1,0 | 0,5 |
| Aromaöl | 1,15 | 1,15 | 1,0 | 1,0 |
| NaOH | 0,158 | 0,158 | 0,158 | 0,158 |
| Wasser | ad 100 | ad 100 | ad 100 | ad 100 |
| Viskosität (20°C, Brookfield, Type RVF Helipath,Spindel TE,4 UpM;nach 1Std.) | 80 Pa•s | 70 Pa•s | 75 Pa•s | 75 Pa•s |

| | 23 | 24 | 25 | 26 | 27 | 28 | 29 | 30 |
|---|---|---|---|---|---|---|---|---|
| Sident 8 | 12,0 | 12,0 | 12,0 | 12,0 | 12,0 | 12,0 | 12,0 | 12,0 |
| Sorbit 70%-ig | 30,0 | 30,0 | 30,0 | 30,0 | 30,0 | 30,0 | 30,0 | 30,0 |
| Glycerin 86%-ig | 33,0 | 33,0 | 33,0 | 33,0 | 33,0 | 33,0 | 33,0 | 33,0 |
| Lipoxol 1550 | 1,0 | 1,0 | 1,0 | 1,0 | 1,0 | 1,0 | 1,0 | 1,0 |
| Na-Saccharinat | 0,2 | 0,2 | 0,2 | 0,2 | 0,2 | 0,2 | 0,2 | 0,2 |
| Na-Benzoat | 0,5 | 0,5 | 0,5 | 0,5 | 0,5 | 0,5 | 0,5 | 0,5 |
| Na2 HPO4 | 0,5 | 0,5 | 0,5 | 0,5 | 0,5 | 0,5 | 0,5 | 0,5 |
| Na-Fluorid | 0,32 | 0,32 | 0,32 | 0,32 | 0,32 | 0,32 | 0,32 | 0,32 |
| MgS04 7H2O | 0,21 | 0,21 | 0,21 | 0,21 | 0,21 | 0,21 | 0,21 | 0,21 |
| ZnSO4 7H2O | 0,1 | 0,1 | 0,1 | 0,1 | 0,1 | 0,1 | 0,1 | 0,1 |
| MnSO4 7H2O | 0,01 | 0,01 | 0,01 | 0,01 | 0,01 | 0,01 | 0,01 | 0,01 |
| Triclosan | 0,2 | 0,2 | 0,2 | 0;2 | 0;2 | 0;2 | 0;2 | 0;2 |
| Xanthan-Gum | 0,55 | 0,55 | 0,55 | 0,55 | 0,55 | 0,55 | 0,55 | 0,55 |
| Parfüm | 1,0 | 1,0 | 1,0 | 1,0 | 1,0 | 1,0 | 1,0 | 1,0 |
| Texapon® K1296 | 1,5 | 2,5 | 1,5 | 1,5 | 1,5 | 1,5 | 1,5 | 1,5 |
| Tagat® S | 0,5 | 0,5 | 0,5 | 1,5 | 0,5 | 0,5 | 0,5 | 0,5 |
| Tego® Betain BL215 | 0,6 | 0,6 | 3,8 | 0,6 | 0,6 | 0,6 | 0,6 | 0,6 |
| Gluadin® W40 | --- | --- | --- | --- | 1,0 | --- | --- | --- |
| Gluadin® Almond | --- | --- | --- | --- | --- | 1,0 | --- | --- |
| Gluadin® R | --- | --- | --- | --- | --- | --- | 1,0 | --- |
| Gluadin® WK | --- | --- | --- | --- | --- | --- | --- | 1,0 |
| Ethanol | 2,0 | 2,0 | 2,0 | 2,0 | 2,0 | 2,0 | 2,0 | 2,0 |
| Wasser | ad 100 | ad 100 | ad 100 | ad 100 | ad 100 | ad 100 | ad 100 | ad 100 |

Es wurden folgende Handelsprodukte verwendet:

| | |
|---|---|
| Plantacare^{®}1200 | C₁₂-C₁₆-Alkylglycosid (Cognis) |
| Sident^{®}8 | Synth. amorph. Kieselsäure (Degussa) |
| Sident^{®}22 S | Hydrogelkieselsäure (Degussa) |
| Polywachs^{®} 1550 | Polyethylenglycol 1550 (RWE/DEA) |
| Texapon^{®}K 1296 | Natrium-Laurylsulfat (Cognis) |
| Cekol^{®}500 T | Natrium-Carboxymethylcellulose (Noviant) |
| Tagat^{®} S | Polyoxyethylen-(20)-glycerylmonostearat (Goldschmidt) |
| Sorbosil® AC 39 | Silica-Abrasiv (Crosfield) |
| Zeodent® 113 | Hydrated Silica (Huber) |
| Zeodent® 623 | Hydrated Silica (Huber) |
| Cremophor® RH 60 | Polyoxyl 60 hydrogenated Castoroil (BASF) |
| Arlatone® 289 | PEG-54 hydrogenated Castoroil (Uniqema) |
| Precarb 100 | Calciumcarbonat |
| Timiron® Diamond Cluster MP 149 | Titandioxid (Merck) |
| Keltrol® F | Xanthan Gum (Keltrol) |
| Tego Betain® BL 215 | Cocamidopropyl betaine (Goldschmidt) |
| Lipoxol® 1550 | Polyethylenglykol 1550 (Sasol) |
| Tagat® S | PEG-20 Glyceryl stearate (Degussa) |
| Gluadin® W40 | Hydrolyzed Wheat Protein (Cognis) |
| Gluadin® Almond | Hydrolyzed Sweet Almond Protein (Cognis) |
| Gluadin® R | Hydrolyzed Rice Protein (Cognis) |
| Gluadin® WK | Sodium cocoyl Hydrolyzed Wheat Protein (Cognis) |

## Patentansprüche

1. Mund- und Zahnpflege- und -reinigungsmittel, enthaltend mindestens ein Enzym ausgewählt aus der Gruppe bestehend aus
- β-Glucanase aus Bacillus amyloliquefaciens,
und
- bezogen auf sein Gewicht - 1 bis 30 Gew.% mindestens eines Putzkörpers.

2. Mund- und Zahnpflege- und -reinigungsmittel nach Anspruch 1, **dadurch gekennzeichnet, dass** genau eines der in Anspruch 1 genannten Enzyme als einziges Enzym im Mittel enthalten ist.

3. Mund- und Zahnpflege- und -reinigungsmittel nach Anspruch 1, **dadurch gekennzeichnet, dass** eine Mischung aus genau zwei der in Anspruch 1 genannten Enzyme im Mittel enthalten ist.

4. Mund- und Zahnpflege- und -reinigungsmittel nach einem der Ansprüche 1 bis 3, enthaltend, jeweils bezogen auf sein Gewicht, 15 bis 35 Gew.-% Wasser und 35 bis 55 Gew.-% mindestens eines mehrwertigen Alkohols aus der Gruppe Sorbit und/oder Glycerin und/oder 1,2-Propylenglycol.

5. Mund- und Zahnpflege- und -reinigungsmittel gemäß Anspruch 4, **dadurch gekennzeichnet, dass** es 20 bis 34 Gew.-%, vorzugsweise 22,5 bis 33 Gew.%, besonders bevorzugt 24 bis 32 Gew.-% und insbesondere 25 bis 31 Gew.% Wasser enthält.

6. Mund- und Zahnpflege- und -reinigungsmittel gemäß Anspruch 4 oder 5, **dadurch gekennzeichnet, dass** es 37,5 bis 52,5 Gew.-%, vorzugsweise 39 bis 51 Gew.%, besonders bevorzugt 40 bis 50 Gew.-% und insbesondere 42 bis 49 Gew.-% mindestens eines mehrwertigen Alkohols aus der Gruppe Sorbit und/oder Glycerin und/oder 1,2-Propylenglycol enthält.

7. Mund- und Zahnpflege- und -reinigungsmittel gemäß einem der Ansprüche 1 bis 6, **dadurch gekennzeichnet, dass** die Enzymaktivität pro Gramm Mund- und Zahnpflege- und - reinigungsmittel 0,05 bis 1500 U, vorzugsweise 0,1 bis 1200 U, besonders bevorzugt 0,25 bis 1000 U und insbesondere 0,5 bis 500 U beträgt.

8. Mund- und Zahnpflege- und -reinigungsmittel gemäß einem der Ansprüche 1 bis 7, **dadurch gekennzeichnet, dass** es Putzkörper aus der Gruppe der Kieselsäuren, Aiuminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper,vorzugsweise in Mengen von 2,5 bis 25 Gew.% und insbesondere von 5 bis 22 Gew.%, jeweils bezogen auf das gesamte Mittel, enthält.

9. Mund- und Zahnpflege- und -reinigungsmittel nach einem der Ansprüche 1 bis 8, **dadurch gekennzeichnet, dass** es zusätzlich Phosphat(e), vorzugsweise Alkalimetallphosphat(e) und insbesondere Natriumtripolyphoyphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthält.

10. Mund- und Zahnpflege- und -reinigungsmittel nach einem der Ansprüche 1 bis 9, **dadurch gekennzeichnet, dass** es zusätzlich Antiplaque-Wirkstoffe, vorzugsweise p-Hydroxybenzoesäuremethyl-, -ethyl- oder propylester, Natriumsorbat, Natriumbenzoat, Bromchlorophen, Triclosan, Phenyl- Salicylsäureester, Biguanide z. B. Chlorhexidin, Thymol, vorzugsweise in Mengen von 0,1 bis 5 Gew.%, vorzugsweise von 0,25 bis 2,5 Gew.% und insbesondere von 0,5 bis 1,5 Gew.%, jeweils bezogen auf das gesamte Mittel, enthält.

11. Mund- und Zahnpflege- und -reinigungsmittel nach einem der Ansprüche 1 bis 10, **dadurch gekennzeichnet, dass** es zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise von 0,1 bis 2,5 Gew.% und insbesondere von 0,2 bis 1,1 Gew.%, jeweils bezogen auf das gesamte Mittel, enthält.

12. Mund- und Zahnpflege- und -reinigungsmittel nach einem der Ansprüche 1 bis 11, **dadurch gekennzeichnet, dass** es die Unempfindlichkeit der Zähne steigende Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 2,5 bis 10 Gew.-% und insbesondere von 4,0 bis 8,0 Gew.%, jeweils bezogen auf das gesamte Mittel, enthält.

13. Mund- und Zahnpflege- und -reinigungsmittel nach einem der Ansprüche 1 bis 12, **dadurch gekennzeichnet, dass** es 0,2 bis 20 Gew.-%, vorzugsweise 0,4 bis 14 Gew.-%, besonders bevorzugt 0,5 bis 3 Gew.-% und insbesondere 0,6 bis 2 Gew.-% mindestens eines bioaktiven Glases enthält.

## Claims

1. An oral and dental care preparation and cleaning agent, containing at least one enzyme selected from the group consisting of
- β-glucanase from *Bacillus amyloliquefaciens,*
and,
- relative to the weight thereof, 1 to 30 wt.% of at least one abrasive cleanser.

2. An oral and dental care preparation and cleaning agent according to claim 1, **characterised in that** exactly one of the enzymes stated in claim 1 stated is present as the sole enzyme in the agent.

3. An oral and dental care preparation and cleaning agent according to claim 1, **characterised in that** a mixture of exactly two of the enzymes stated in claim 1 is present in the agent.

4. An oral and dental care preparation and cleaning agent according to any one of claims 1 to 3, containing, in each case relative to the weight thereof, 15 to 35 wt.% of water and 35 to 55 wt.% of at least one polyhydric alcohol from the group sorbitol and/or glycerol and/or 1,2-propylene glycol.

5. An oral and dental care preparation and cleaning agent according to claim 4, **characterised in that** it contains 20 to 34 wt.%, preferably 22.5 to 33 wt.%, more preferably 24 to 32 wt.% and in particular 25 to 31 wt.% of water.

6. An oral and dental care preparation and cleaning agent according to claim 4 or 5, **characterised in that** it contains 37.5 to 52.5 wt.%, preferably 39 to 51 wt.%, more preferably 40 to 50 wt.% and in particular 42 to 49 wt.% of at least one polyhydric alcohol from the group of sorbitol and/or glycerol and/or 1,2-propylene glycol.

7. An oral and dental care preparation and cleaning agent according to any one of claims 1 to 6, **characterised in that** the enzyme activity pro gram of oral and dental care preparation and cleaning agent amounts to 0.05 to 1500 U, preferably 0.1 to 1200 U, more preferably 0.25 to 1000 U and in particular 0.5 to 500 U.

8. An oral and dental care preparation and cleaning agent according to any one of claims 1 to 7, **characterised in that** it contains abrasive cleansers from the group of silicas, aluminium hydroxide, aluminium oxide, calcium pyrophosphate, chalk, dicalcium phosphate dihydrate (CaHPO₄-2H₂O), sodium-aluminium silicates, in particular zeolite A, organic polymers, in particular polymethacrylates or mixtures of these abradants, preferably in quantities of 2.5 to 25 wt % and in particular of 5 to 22 wt.%, in each case relative to the entire agent.

9. An oral and dental care preparation and cleaning agent according to any one of claims 1 to 8, **characterised in that** it additionally contains phosphate(s), preferably alkali metal phosphate(s) and in particular sodium tripolyphosphate, preferably in quantities of 1 to 10 wt.%, more preferably of 2 to 8 wt.% and in particular of 3 to 7 wt.%, in each case relative to the entire agent.

10. An oral and dental care preparation and cleaning agent according to any one of claims 1 to 9, **characterised in that** it additionally contains antiplaque active substances, preferably p-hydroxybenzoic acid methyl, ethyl or propyl ester, sodium sorbate, sodium benzoate, bromochlorophen, triclosan, phenyl salicylate ester, biguanides for example chlorhexidine, thymol, preferably in quantities of 0.1 to 5 wt.%, preferably of 0.25 to 2.5 wt.% and in particular of 0.5 to 1.5 wt.%, in each case relative to the entire agent.

11. An oral and dental care preparation and cleaning agent according to any one of claims 1 to 10, **characterised in that** it additionally contains anticaries active substances, preferably fluorine compound(s), in particular sodium fluoride, potassium fluoride, sodium monofluorophosphate, zinc fluoride, stannous fluoride and sodium fluorosilicate, preferably in quantities of 0.01 to 5 wt.%, preferably of 0.1 to 2.5 wt.% and in particular of 0.2 to 1.1 wt.%, in each case relative to the entire agent.

12. An oral and dental care preparation and cleaning agent according to any one of claims 1 to 11, **characterised in that** it contains substances which decrease dental sensitivity, preferably potassium salts, more preferably potassium nitrate and/or potassium citrate and/or potassium chloride and/or potassium bicarbonate and/or potassium oxalate, preferably in quantities of 0.5 to 20 wt.%, more preferably of 1.0 to 15 wt.%, further preferably of 2.5 to 10 wt.% and in particular of 4.0 to 8.0 wt.%, in each case relative to the entire agent.

13. An oral and dental care preparation and cleaning agent according to any one of claims 1 to 12, **characterised in that** it contains 0.2 to 20 wt.%, preferably 0.4 to 14 wt.%, more preferably 0.5 to 3 wt.% and in particular 0.6 to 2 wt.% of at least one bioactive glass.

## Revendications

1. Agent de soins et de nettoyage buccal et dentaire contenant au moins une enzyme choisie parmi le groupe constitué par
- des β-glucanases issues de Bacillus amyloliquefaciens ;
et
- rapportés à son poids, de 1 à 30 % en poids d'au moins un corps de brossage.

2. Agent de soins et de nettoyage buccal et dentaire selon la revendication 1, **caractérisé en ce qu'**il contient précisément une des enzymes mentionnées à la revendication 1 à titre d'enzyme unique dans l'agent.

3. Agent de soins et de nettoyage buccal et dentaire selon la revendication 1, **caractérisé en ce qu'**il contient un mélange de précisément deux des enzymes mentionnées à la revendication 1 dans l'agent.

4. Agent de soins et de nettoyage buccal et dentaire selon l'une quelconque des revendications 1 à 3, contenant, chaque fois rapportés à son poids, de 15 à 35 % en poids d'eau et de 35 à 55 % en poids d'au moins un alcool polyvalent choisi parmi le groupe du sorbitol et/ou du glycérol et/ou du 1,2-propylèneglycol.

5. Agent de soins et de nettoyage buccal et dentaire selon la revendication 4, **caractérisé en ce qu'**il contient de 20 à 34 % en poids, de préférence de 22,5 à 33 % en poids, de manière particulièrement préférée de 24 à 32 % en poids et en particulier de 25 à 31 % en poids d'eau.

6. Agent de soins et de nettoyage buccal et dentaire selon la revendication 4 ou 5, **caractérisé en ce qu'**il contient de 37, 5 à 52, 5 % en poids, de préférence de 39 à 51 % en poids, de manière particulièrement préférée de 40 à 50 % en poids et en particulier de 42 à 49 % en poids d'au moins un alcool polyvalent choisi parmi le groupe du sorbitol et/ou du glycérol et/ou du 1,2-propylèneglycol.

7. Agent de soins et de nettoyage buccal et dentaire selon l'une quelconque des revendications 1 à 6, **caractérisé en ce que** l'activité enzymatique par gramme de l'agent de soins et de nettoyage buccal et dentaire s'élève de 0,05 à 1500 U, de préférence de 0,1 à 1200 U, de manière particulièrement préférée de 0,25 à 1000 U, et en particulier de 0,5 à 500 U.

8. Agent de soins et de nettoyage buccal et dentaire selon l'une quelconque des revendications 1 à 7, **caractérisé en ce qu'**il contient des corps de brossage choisis parmi le groupe des acides siliciques, de l'hydroxyde d'aluminium, de l'oxyde d'aluminium, du pyrophosphate de calcium, de la craie, du dihydrate de phosphate dicalcique (CaHPO₄·2H₂O), des silicates de sodium-aluminium, en particulier la zéolithe A, des polymères organiques, en particulier des polyméthacrylates, ou des mélanges de ces corps de friction, de préférence dans des quantités de 2,5 à 25 % en poids et en particulier de 5 à 22 % en poids, chaque fois rapportés à l'agent dans sa totalité.

9. Agent de soins et de nettoyage buccal et dentaire selon l'une quelconque des revendications 1 à 8, **caractérisé en ce qu'**il contient en outre un ou plusieurs phosphates, de préférence un ou plusieurs phosphates de métaux alcalins et en particulier du tripolyphosphate de sodium, de préférence dans des quantités de 1 à 10 % en poids, de manière particulièrement préférée de 2 à 8 % en poids et en particulier de 3 à 7 % en poids, chaque fois rapportés à l'agent dans sa totalité.

10. Agent de soins et de nettoyage buccal et dentaire selon l'une quelconque des revendications 1 à 9, **caractérisé en ce qu'**il contient en outre des substances actives s'opposant à la formation de la plaque, de préférence des esters méthyliques, éthyliques ou propyliques de l'acide p-hydroxybenzoïque, le sorbate de sodium, le benzoate de sodium, le bromochlorophen, le triclosan, des esters phényliques de l'acide salicylique, des biguanides, par exemple la chlorhexidine, le thymol, de préférence dans des quantités de 0,1 à 5 % en poids, de préférence de 0,25 à 2,5 % en poids et en particulier de 0,5 à 1,5 % en poids, chaque fois rapportés à l'agent dans sa totalité.

11. Agent de soins et de nettoyage buccal et dentaire selon l'une quelconque des revendications 1 à 10, **caractérisé en ce qu'**il contient en outre des substances actives s'opposant à la formation de caries, de préférence un ou plusieurs composés de fluor, en particulier le fluorure de sodium, le fluorure de potassium, le monofluorophosphate de sodium, le fluorure de zinc, le fluorure d'étain et le fluorosilicate de sodium, de préférence dans des quantités de 0,01 à 5 % en poids, de préférence de 0,1 à 2,5 % en poids et en particulier de 0,2 à 1,1 % en poids, chaque fois rapportés à l'agent dans sa totalité.

12. Agent de soins et de nettoyage buccal et dentaire selon l'une quelconque des revendications 1 à 11, **caractérisé en ce qu'**il contient des substances qui augmentent l'insensibilité des dents, de préférence des sels de potassium, de manière particulièrement préférée du nitrate de potassium et/ou du citrate de potassium et/ou du chlorure de potassium et/ou du bicarbonate de potassium et/ou de l'oxalate de potassium, de préférence dans des quantités de 0,5 à 20 % en poids, de manière particulièrement préférée de 1,0 à 15 % en poids, de manière plus préférée de 2,5 à 10 % en poids et en particulier de 4,0 à 8,0 % en poids, chaque fois rapportés à l'agent dans sa totalité.

13. Agent de soins et de nettoyage buccal et dentaire selon l'une quelconque des revendications 1 à 12, **caractérisé en ce qu'**il contient de 0,2 à 20 % en poids, de préférence de 0,4 à 14 % en poids, de manière particulièrement préférée de 0,5 à 3 % en poids et en particulier de 0,6 à 2 % en poids d'au moins un verre bioactif.
